(19) Europäisches Patentamt
European Patent Office
Office européen des brevets

(11) Veröffentlichungsnummer: **0 125 207**
**A1**

(12) **EUROPÄISCHE PATENTANMELDUNG**

(21) Anmeldenummer: **84810204.2**

(22) Anmeldetag: **30.04.84**

(51) Int. Cl.³: **C 07 D 499/00**
A 61 K 31/43
//C07F7/18, C07D257/04, C07D205/08, C07F9/65

(30) Priorität: **06.05.83 CH 2490/83**

(43) Veröffentlichungstag der Anmeldung:
**14.11.84 Patentblatt 84/46**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Lang, Marc, Dr.**
**Rue des Ormes 6**
**F-68170 Rixheim(FR)**

(54) **Heterocyclylniederalkyl-penem-Verbindungen, Verfahren zu ihrer Herstellung, pharmazeutische Präparate, welche diese Verbindungen enthalten, und Verwendung von letzteren.**

(57) 2-Heterocyclylniederalkyl-2-penem-Verbindungen der Formel

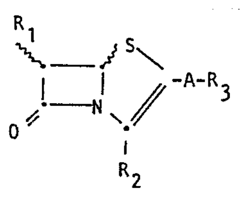

(I),

worin $R_1$ durch Hydroxy oder geschütztes Hydroxy substituiertes Niederalkyl ist, $R_2$ Carboxyl oder funktionell abgewandeltes Carboxyl bedeutet, $R_3$ einen über ein tertiäres Ringstickstoffatom an den Rest –A– gebundenen, ungesättigten monocyclischen Azaheterocyclyl-Rest darstellt und A durch Niederalkyl substituiertes geradkettiges Niederalkylen bedeutet, optische Isomere von Verbindungen der Formel (I), Mischungen dieser optischen Isomere und Salze von solchen Verbindungen der Formel (I), die eine salzbildende Gruppe aufweisen besitzen antibiotische Eigenschaften. Die neuen Verbindungen können z.B. in Form von antibiotisch wirksamen Präparaten zur Behandlung von Infektionskranheiten verwendet werden. Die neuen Verbindungen können in an sich bekannter Weise hergestellt werden.

EP 0 125 207 A1

0125207

CIBA-GEIGY AG
Basel (Schweiz)

4-14416/=

Heterocyclylniederalkyl-penem-Verbindungen, Verfahren zu ihrer Herstellung, pharmazeutische Präparate, welche diese Verbindungen enthalten, und Verwendung von letzteren.

Die vorliegende Erfindung betrifft neue 2-Heterocyclylniederalkylpenem-Verbindungen, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere 2-Heterocyclylniederalkyl-2-penem-Verbindungen der Formel

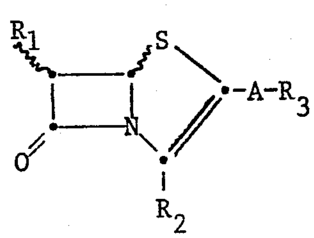

(I),

worin $R_1$ durch Hydroxy oder geschütztes Hydroxy substituiertes Niederalkyl ist, $R_2$ Carboxyl oder funktionell abgewandeltes Carboxyl bedeutet, $R_3$ einen über ein tertiäres Ringstickstoffatom an den Rest -A- gebundenen, ungesättigten monocyclischen Azaheterocyclyl-Rest darstellt und A durch Niederalkyl substituiertes geradkettiges Niederalkylen bedeutet, optische Isomere von Verbindungen der Formel (I), Mischungen dieser optischen Isomere und Salze von solchen Verbindungen der Formel (I), die eine salzbildende Gruppe aufweisen.

Die vor- und nachstehend verwendeten Definitionen haben im Rahmen der vorliegenden Beschreibung vorzugsweise die folgenden Bedeutungen:

Funktionell abgewandeltes Carboxyl $R_2$ ist insbesondere unter physiologischen Bedingungen spaltbares, verestertes Carboxyl oder geschütztes Carboxyl $R_2'$.

Eine unter physiologischen Bedingungen spaltbare veresterte Carboxylgruppe $R_2$ schützt die Verbindungen der Formel I vor Salzbildung im Magen-Darm-Trakt bei oraler Verabreichung, womit die vorzeitige Exkretion verhindert wird, und ist in erster Linie eine Acyloxymethoxycarbonylgruppe, worin Acyl z.B. den Rest einer organischen Carbonsäure, in erster Linie einer gegebenenfalls substituierten Niederalkancarbonsäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere α-Aminoniederalkanoyloxymethoxycarbonyl, 4-Crotonolactonyl und 4-Butyrolacton-4-yl. Weitere unter physiologischen Bedingungen spaltbare veresterte Carboxylgruppen $R_2$ sind z.B. 5-Indanyloxycarbonyl, 3-Phthalidyloxycarbonyl, 1-Niederalkoxycarbonyloxyniederalkoxycarbonyl, 1-Niederalkoxyniederalkoxycarbonyl oder 2-Oxo-1,3-dioxolen-4-ylmethoxycarbonyl, welches in 5-Stellung des Dioxolenringes durch Niederalkyl oder Phenyl substituiert ist.

Ein über ein tertiäres Ringstickstoffatom an den Rest -A- gebundener, ungesättigter monocyclischer Azaheterocyclyl-Rest $R_3$ ist insbesondere ein entsprechender gegebenenfalls partiell gesättigter 5-gliedriger Heteroaryl-Rest mit 1 bis 4 Ringstickstoffatomen, wie ein entsprechender aza-, diaza-, triaza- oder tetraza-cyclischer Rest aromatischen Charakters oder ein entsprechender Dihydro-Rest, oder ein entsprechender partiell gesättigter 6-gliedriger Heteroaryl-Rest mit 1 bis 3 Ringstickstoffatomen, wie ein entsprechender aza-, diaza- oder triaza-cyclischer Rest, z.B. ein entsprechender

Dihydro- oder Tetrahydro-Rest. Entsprechende Reste $R_3$ sind z.B. gegebenenfalls partiell gesättigtes Pyrrolyl, Diazolyl, Triazolyl oder Tetrazolyl oder partiell gesättigtes Pyridyl, Pyrimidyl, Pyridazinyl, Pyrazinyl oder Triazinyl. Diese Reste sind unsubstituiert oder können durch gegebenenfalls veräthertes oder verestertes, inklusive geschützes Hydroxy, z.B. Hydroxy, Niederalkoxy, Niederalkanoyloxy oder Halogen, gegebenenfalls veräthertes Mercapto, z.B. Mercapto, Niederalkylthio oder Phenylthio, Niederalkyl, Hydroxyniederalkyl, Niederalkoxyniederalkyl, Carboxyniederalkyl, gegebenenfalls N-niederalkyliertes Aminoniederalkyl, z.B. Aminoniederalkyl oder Diniederalkylaminoniederalkyl, Sulfoniederalkyl, gegebenenfalls substituiertes, inklusive geschütztes Amino, z.B. Amino, Niederalkylamino, Diniederalkylamino, Niederalkylenamino oder Acylamino, wie Niederalkanoylamino, gegebenenfalls funktionell abgewandeltes, inklusive geschütztes Carboxyl oder Sulfo, z.B. Carboxyl, Sulfo, verestertes Carboxyl, wie Niederalkoxycarbonyl, gegebenenfalls substituiertes Carbamoyl, wie N-mono- oder N,N-diniederalkyliertes Carbamoyl, Cyano oder Sulfamoyl, gegebenenfalls durch Niederalkyl, Nitro, Niederalkoxy und/oder Halogen substituiertes Phenyl, Cycloalkyl, Nitro, Oxo und/oder Oxido substituiert, wie insbesondere mono- oder auch polysubstituiert, wie insbesondere mono- oder disubstituiert, sein.

In der vorliegenden Beschreibung bedeutet der im Zusammenhang mit Definitionen von Gruppen bzw. Verbindungen verwendete Ausdruck "Nieder", dass die entsprechenden Gruppen bzw. Verbindungen, sofern nicht ausdrücklich anders definiert, bis zu 7, bevorzugt bis zu 4 Kohlenstoffatome enthalten.

Durch Hydroxy substituiertes Niederalkyl $R_1$ ist insbesondere in α-Stellung zum Penem-Ringgerüst durch Hydroxy substituiertes Niederalkyl und bedeutet z.B. 1-Hydroxy-prop-1-yl, 2-Hydroxy-prop-2-yl, 1-Hydroxybut-1-yl, 2-Hydroxybut-2-yl oder insbesondere

Hydroxymethyl oder 1-Hydroxyäthyl.

Niederalkanoyloxymethoxycarbonyl ist z.B. Acetoxymethoxycarbonyl oder Pivaloyloxymethoxycarbonyl.

α-Aminoniederalkanoyloxymethoxycarbonyl ist z.B. Glycyloxymethoxycarbonyl, Valyloxymethoxycarbonyl oder Leucyloxymethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Niederalkoxy ist z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy.

Niederalkanoyloxy ist z.B. Acetyloxy oder Propionyloxy.

Halogen ist z.B. Fluor, Chlor, Brom oder Jod.

Niederalkylthio ist z.B. Methylthio, Aethylthio, n-Propylthio, Isopropylthio oder n-Butylthio.

Niederalkyl als Substituent eines Restes $R_3$ ist z.B. Methyl, Aethyl, n-Propyl, Isopropyl, n-Butyl, Isobutyl, sek.-Butyl oder tert.-Butyl, ferner n-Pentyl, n-Hexyl oder n-Heptyl.

Hydroxyniederalkyl als Substituent eines Restes $R_3$ ist z.B. Hydroxymethyl, 2-Hydroxyäthyl oder 2,3-Dihydroxypropyl.

Niederalkoxyniederalkyl ist z.B. Methoxymethyl, 2-Methoxyäthyl, Aethoxymethyl oder 2-Aethoxyäthyl.

Carboxyniederalkyl ist z.B. Carboxymethyl, 1-Carboxy-, 2-Carboxy- oder 1,2-Dicarboxyäthyl.

Aminoniederalkyl ist z.B. Aminomethyl oder 2-Aminoäthyl, während Diniederalkylaminoniederalkyl z.B. Dimethylaminomethyl, 2-Dimethylaminoäthyl oder 2-Diäthylaminoäthyl ist.

Sulfoniederalkyl ist z.B. Sulfomethyl oder 2-Sulfoäthyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkylenamino weist insbesondere 4 bis 6 Kohlenstoffkettenglieder auf und bedeutet z.B. Pyrrolidino oder Piperidino.

Niederalkanoylamino ist z.B. Acetylamino oder Propionylamino.

Niederalkoxycarbonyl ist z.B. Methoxycarbonyl oder Aethoxycarbonyl.

N-Mono-niederalkyliertes Carbamoyl ist z.B. N-Methyl-, N-Aethyl-, oder N-Propylcarbamoyl, während N,N-di-niederalkyliertes Carbamoyl z.B. N,N-Dimethyl oder N,N-Diäthylcarbamoyl bedeutet.

Cycloalkyl enthält 3 bis 8, in erster Linie 5 oder 6 Ringglieder und ist z.B. Cyclopentyl oder Cyclohexyl, ferner Cyclopropyl sowie Cycloheptyl.

Bevorzugte, unter physiologischen Bedingungen spaltbare veresterte Carboxylgruppen $R_2$ sind z.B. Phthalidyloxycarbonyl, Niederalkanoyloxymethoxycarbonyl, z.B. Pivaloyloxymethoxycarbonyl, und 1-Niederalkoxycarbonyloxy-niederalkoxycarbonyl, z.B. 1-Aethoxycarbonyloxyäthoxycarbonyl.

Entsprechende 5-gliedrige gegebenenfalls partiell gesättigte Heteroarylreste $R_3$ sind z.B. gegebenenfalls z.B. durch Niederalkyl oder Halogen substituiertes Pyrrolyl oder Dihydropyrrolyl, z.B. 1-Pyrrolyl, 3-Methyl-1-pyrrolyl, 3,4-Dichlor-1-pyrrolyl, ferner 2,3- oder 2,5-Dihydro-1-pyrrolyl, gegebenenfalls z.B. durch Niederalkyl, Aminoniederalkyl, Amino oder Nitro substituiertes Diazolyl, wie Imidazolyl oder Pyrazolyl, z.B. 1-Imidazolyl oder 1-Pyrazolyl, gegebenenfalls z.B. durch Niederalkyl, Carboxyniederalkyl, Amino oder Phenyl substituiertes Triazolyl, wie 1H-1,2,3-Triazol-1-yl, 2H-1,2,3-Triazol-2-yl, 1H-1,2,4-Triazol-1-yl oder 1H-1,3,4-Triazol-1-yl, z.B. die entsprechenden unsubstituierten Reste, 4- oder 5-Methyl-1,2,3-triazol-1-yl, 3-Methyl- oder 3-Phenyl-1H-1,2,4-triazol-1-yl, oder gegebenenfalls z.B. durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolyl, wie 1H-Tetrazol-1-yl oder 2H-Tetrazol-2-yl, z.B. die entsprechenden unsubstituierten Reste, 5-Amino-, 5-Methyl-, 5-Carboxymethyl-, 5-(2-Carboxyäthyl)-, 5-Sulfomethyl-, 5-(2-Dimethylaminoäthyl)- oder 5-Phenyl-1H-tetrazol-1-yl, oder 5-Amino-, 5-Methyl-, 5-Carboxymethyl-, 5-Sulfomethyl-, 5-(2-Dimethylaminoäthyl)- oder 5-Phenyl-2H-tetrazol-2-yl.

Entsprechende 6-gliedrige partiell gesättigte Heteroarylreste $R_3$ sind z.B. unsubstituiertes oder insbesondere z.B. durch Oxo und gegebenenfalls zusätzlich durch Halogen substituiertes Dihydro-1-pyridyl, wie 2H-1,2-Dihydro- oder 4H-1,4-Dihydro-1-pyridyl, z.B. 2-Oxo-2H-1,2-dihydro-1-pyridyl oder 4-Oxo-4H-1,4-dihydro-1-pyridyl, gegebenenfalls, z.B. insbesondere durch Oxo und gegebenenfalls zusätzlich z.B. durch Niederalkyl, Amino, Diniederalkylamino oder Carboxy substituiertes

Dihydro-1-pyrimidyl, wie 2H-1,2-Dihydro- oder 4H-1,4-Dihydro-1-pyrimidyl, z.B. 2-Oxo-1,2-dihydro-1-pyrimidyl, 6-Methyl-, 5-Methyl-, 6-Amino-, 6-Dimethylamino-, 5-Carboxy- oder 6-Carboxy-2-oxo-1,2-dihydro-1-pyrimidyl oder 4-Oxo-1,4-dihydro-1-pyrimidyl, oder gegebenenfalls z.B. durch Niederalkyl und/oder bis zu 2 Oxo substituiertes Dihydro- oder Tetrahydrotriazinyl, z.B. 2H-1,2-Dihydro-1,3,5-triazin-1-yl, 2H-1,2-dihydro-1,2,4-triazin-1-yl, 2H-1,2,5,6-Tetrahydro-1,2,4-triazin-1-yl oder 4H-1,4,5,6-Tetrahydro-1,2,4-triazin-1-yl, z.B. 4-Niederalkyl-1,4,5,6-tetrahydro-5,6-dioxo-1,2,4-triazin-1-yl, z.B. 4-Methyl-1,4,5,6-tetrahydro-5,6-dioxo-1,2,4-triazin-1-yl.

Geradkettiges Niederalkylen im Rest A weist 1-7 und vorzugsweise 1-4 Kohlenstoffatome auf und ist z.B. Methylen, Aethylen, 1,3-Propylen, 1,4-Butylen, ferner auch 1,5-Pentylen. Ein solcher Niederalkylen-Rest ist durch Niederalkyl mit 1-4 Kohlenstoffatomen, insbesondere mit 1 oder 2 Kohlenstoffatomen, wie n-Propyl, n-Butyl oder insbesondere Methyl oder Aethyl, mono- oder polysubstituiert, wie insbesondere mono- oder disubstituiert. Zwei Niederalkylsubstituenten können am gleichen Kohlenstoffatom (geminal), an benachbarten Kohlenstoffatomen (vicinal) oder an verschiedenen, durch mindestens eine Methylengruppe getrennten Kohlenstoffatomen der Niederalkylen-Kette angeordnet sein.

Ein solcher Rest A ist beispielsweise Niederalkyliden, wie Aethyliden ("Methyl-methylen"), 1,2-Propylen, 2-Niederalkyl-, wie 2-Methyl-1,2-propylen, 1,2-Butylen, 2-Niederalkyl-, wie 2-Methyl- oder 2-Aethyl-1,2-butylen, 3-Niederalkyl-, wie 3-Methyl-1,2-butylen, 1,3-Butylen, 2-Niederalkyl-, wie 2-Methyl-1,3-butylen, 3-Niederalkyl-, wie 3-Methyl-1,3-butylen, oder 2,2-Diniederalkyl-, wie 2,2-Dimethyl-1,3-butylen, wobei in diesen Resten das Kohlenstoffatom 1 mit dem Penem-Ringgerüst verbunden ist, oder auch 1,2-Propylen, 2-Niederalkyl-, wie 2-Methyl-1,2-propylen, 1,2-Butylen, 2-Niederalkyl-, wie 2-Methyl- oder 2-Aethyl-1,2-butylen, 3-Niederalkyl-, wie 3-Methyl-1,2-butylen, oder 3,3-Diniederalkyl-, wie 3,3-Dimethyl-1,2-butylen, wobei in diesen Resten das Kohlenstoffatom 2 mit dem Penem-Ringgerüst verbunden ist.

Bevorzugte Reste A sind über das Kohlenstoffatom 1 mit dem Penem-Ringgerüst verbundenes Aethyliden, 1,2-Propylen, 1,2-Butylen, 1,3-Butylen und 2-Methyl-1,2-propylen, sowie über das Kohlenstoffatom 2 mit dem Penem-Ringgerüst verbundenes 1,2-Propylen.

Die in den Verbindungen der Formel I vorhandenen funktionellen Gruppen, wie Hydroxy-, Carboxy-, Amino- oder Sulfogruppen, insbesondere die Hydroxygruppe im Rest $R_1$ und die Carboxylgruppe $R_2$, sind gegebenenfalls durch konventionelle Schutzgruppen geschützt, die in der Penem-, Penicillin-, Cephalosporin- und Peptidchemie verwendet werden.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) kann eine Hydroxygruppe im Rest $R_1$, ferner eine im Rest $R_3$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl- oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, oder gegebenenfalls durch Nitro substituiertes Phenylniederalkoxycarbonyl, z.B. 4-Nitrobenzyloxycarbonyl. Weitere geeignete Hydroxyschutzgruppen sind z.B. trisubstituiertes Silyl, wie Triniederalkylsilyl, z.B. Trimethylsilyl oder tert.-Butyl-dimethylsilyl, 2-Halogenniederalkylgruppen, z.B. 2-Chlor-, 2-Brom-, 2-Jod- und 2,2,2-Trichloräthyl, und gegebenenfalls durch Halogen, z.B. Chlor, Niederalkoxy, z.B. Methoxy, und/oder Nitro substituiertes Phenylniederalkyl, wie entsprechendes Benzyl. Bevorzugt als Hydroxyschutzgruppe ist Triniederalkylsilyl.

Eine Carboxylgruppe $R_2$, ferner auch eine im Rest $R_3$ vorhandene Carboxylgruppe ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist. Eine geschützte Carboxylgruppe kann ferner eine leicht in eine andere funktionell abgewandelte Carboxylgruppe, wie in eine andere veresterte Carboxylgruppe umwandelbare, veresterte Carboxylgruppe darstellen.

Solche veresterten Carboxylgruppen enthalten als veresternde Gruppen in erster Linie in 1-Stellung verzweigte oder in 1- oder 2-Stellung geeignet substituierte Niederalkylgruppen. Bevorzugte in veresterter Form vorliegende Carboxylgruppen sind unter anderen Niederalkoxycarbonyl, z.B. Methoxycarbonyl, Aethoxycarbonyl, Isopropoxycarbonyl oder tert.-Butoxycarbonyl, und (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls,z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder ω-Halogenniederalkoxycarbonyl, worin Niederalkoxy 4-7 Kohlenstoffatome enthält, z.B. 4-Chlorbutoxycarbonyl, Phthalimidomethoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl.

Weitere in veresterter Form vorliegende geschützte Carboxylgruppen sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Aethyl, ferner Niederalkoxy, z.B. Methoxy, als Substituenten. Geeignete Silyl- bzw. Stannylschutzgruppen sind in erster Linie Triniederalkylsilyl, insbesondere Trimethylsilyl oder Dimethyl-tert.-butylsilyl, oder entsprechend substituierte Stannylgruppen, z.B. Tri-n-butylstannyl.

Bevorzugte geschütze Carboxylgruppen sind die 4-Nitrobenzyloxycarbonyl-, Niederalkenyloxycarbonyl- oder die in 2-Stellung durch Triniederalkylsilyl substituierte Aethoxycarbonylgruppe.

Eine geschütze Aminogruppe kann beispielsweise in Form einer leicht spaltbaren Acylamino-, Acylimino-, verätherten Mercaptoamino-, Silyl- oder Stannylaminogruppe oder als Enamino-, Nitro- oder Azidogruppe vorliegen.

In einer entsprechenden Acylaminogruppe ist Acyl beispielsweise der Acylrest einer organischen Säure mit z.B. bis zu 18 Kohlenstoffatomen,

insbesondere einer gegebenenfalls, z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Actyl oder Propionyl, Halogenniederalkanoyl, wie 2-Halogenacetyl, insbesonder 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1- oder 2-Stellung substituiertes Niederalkoxycarbonyl geeignet, wie Niederalkoxycarbonyl, z.B. Methoxy- oder Aethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trissubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl, oder 2-Triarylsilyläthoxycarbonyl, wie 2-Triphenylsilyläthoxycarbonyl.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält. Entsprechende Silyl- oder Stannylgruppen sind in erster Linie Triniederalkylsilyl, insbesondere Trimethylsilyl, ferner Dimethyl-tert.-butylsilyl, oder entsprechend substituiertes Stannyl, z.B. Tri-n-butylstannyl.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Schutzgruppen dieser Art sind beispielsweise 1-Acyl-niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -äthylhalbesters, und Niederalk-1-en insbesondere 1-Propen bedeutet. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine geschütze Sulfogruppe im Rest $R_3$ ist in erster Linie eine veresterte, wie mit einem aliphatischen, cycloaliphatischen, cycloaliphatisch-aliphatischen, aromatischen oder araliphatischen Alkohol, z.B. einem Niederalkanol, oder mit einem Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxylgruppe veräthert sein.

Salze von erfindungsgemässen Verbindungen sind in erster Linie pharmazeutisch annehmbare, nicht-toxische Salze von Verbindungen der Formel I. Solche Salze werden beispielsweise von den in Verbindungen

der Formel I vorhandenen sauren Gruppen, z.B. Carboxyl- und Sulfogruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethylpiperidin, Cycloalkylaminen, z.B. Dicyclohexylamin, oder Benzylaminen, z.B. N,N'-Dibenzyläthylendiamin, Dibenzylamin oder N-Benzyl-β-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salzen, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

In den Penemverbindungen der Formel I können die beiden asymmetrischen Kohlenstoffatome in 5- und 6-Stellung in der R-, der S- oder racemischen R,S-Konfiguration vorliegen. Bevorzugt sind die Verbindungen, in denen die Konfiguration des 5-Kohlenstoffatoms derjenigen des natürlichen Penicillins enspricht (5R-Konfiguration). Die Wasserstoffatome in 5- und 6-Stellung können in cis- oder bevorzugt in trans-Stellung zueinander stehen. In der bevorzugten Konfiguration nimmt der Substituent in 6-Stellung die S-Konfiguration

ein. Die Verbindungen der Formel I können in den Resten $R_1$ und/oder A weitere Chiralitätszentren besitzen, welche jeweils in der R-, S- oder in der R,S-Konfiguration vorliegen können. In α-Stellung durch Hydroxy substituiertes Niederalkyl $R_1$ weist bevorzugt die R-Konfiguration auf.

Die Erfindung betrifft insbesondere Verbindungen der Formel I, worin $R_1$ durch Hydroxy oder geschütztes Hydroxy substituiertes Niederalkyl ist, $R_2$ Carboxyl, unter physiologischen Bedingungen spaltbares, verestertes Carboxyl oder geschütztes Carboxyl $R_2'$ bedeutet, $R_3$ einen über ein tertiäres Ringstickstoffatom an den Rest -A- gebundenen monocyclischen, gegebenenfalls partiell gesättigten 5-gliedrigen Heteroaryl-Rest mit 1-4 Ringstickstoffatomen, wie einen entsprechenden aza-, diaza-, triaza- oder tetraza-cyclischen Rest aromatischen Charakters oder einen entsprechenden Dihydro-Rest, oder einen entsprechenden partiell gesättigten 6-gliedrigen Heteroaryl-Rest mit 1-3 Ringstickstoffatomen, wie einen entsprechenden aza-, diaza- oder triaza-cyclischen Rest, z.B. einen entsprechenden Dihydro- oder Tetrahydro-Rest, darstellt, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, Aminoniederalkyl, Diniederalkylaminoniederalkyl, Sulfoniederalkyl, Amino, Niederalkylamino, Diniederalkylamino, Niederalkylenamino, Niederalkanoylamino, Carboxy, Niederalkoxycarbonyl, gegebenenfalls N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Oxo und/oder Oxido substituiert sind, und A durch Niederalkyl substituiertes geradkettiges Niederalkylen bedeutet, optische Isomere von Verbindungen der Formel (I), Mischungen dieser optischen Isomere und Salze von Verbindungen der Formel (I), die eine salzbildende Gruppe aufweisen.

Die Erfindung betrifft vor allem Verbindungen der Formel (I), worin $R_1$ durch Hydroxy oder Triniederalkylsilyloxy substituiertes Nieder-

alkyl ist, $R_2$ Carboxyl, Niederalkenyloxycarbonyl, gegebenenfalls durch Nitro substituiertes Benzyloxycarbonyl, 2-Triniederalkylsilyläthoxycarbonyl oder eine unter physiologischen Bedingungen spaltbare veresterte Carboxylgruppe, z.B. 1-Niederalkoxycarbonyloxyniederalkoxycarbonyl, Niederalkanoyloxymethoxycarbonyl, α-Aminoniederalkanoyloxymethoxycarbonyl oder Phthalidyloxycarbonyl, bedeutet, $R_3$ über ein tertiäres Stickstoffatom an den Rest -A- gebundenes, unsubstituiertes oder durch Niederalkyl oder Halogen substituiertes Pyrrolyl, z.B. 1-Pyrrolyl, unsubstituiertes oder durch Niederalkyl substituiertes Imidazolyl oder Pyrazolyl, z.B. 1-Imidazolyl oder 1-Pyrazolyl, unsubstituiertes oder durch Niederalkyl, Carboxyniederalkyl oder Phenyl substituiertes Triazolyl, z.B. 1H-1,2,3-Triazol-1-yl, 2H-1,2,3-Triazol-2-yl, 1H-1,2,4-Triazol-1-yl oder 1H-1,3,4-Triazol-1-yl, unsubstituiertes oder durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolyl, wie 1H-Tetrazol-1-yl oder 2H-Tetrazol-2-yl, unsubstituiertes oder durch Oxo und gegebenenfalls zusätzlich durch Halogen substituiertes Dihydro-1-pyridyl, z.B. 2H-1,2-Dihydro-1-pyridyl oder 4H-1,4-Dihydro-1-pyridyl, unsubstituiertes oder durch Oxo und gegebenenfalls zusätzlich durch Niederalkyl, Amino, Diniederalkylamino oder Carboxy substituiertes Dihydro-1-pyrimidyl, z.B. 2H-1,2-Dihydro-1-pyrimidyl oder 4H-1,4-Dihydro-1-pyrimidyl, oder unsubstituiertes oder durch Niederalkyl und/oder bis zu zwei Oxo substituiertes Dihydro- oder Tetrahydrotriazinyl, z.B. 2H-1,2-Dihydro-1,3,5-triazin-1-yl, 2H-1,2-Dihydro-1,2,4-triazin-1-yl, 2H-1,2,5,6-Tetrahydro-1,2,4-triazin-1-yl oder 4H-1,4,5,6-Tetrahydro-1,2,4-triazin-1-yl, bedeutet, und A durch Niederalkyl substituiertes geradkettiges Niederalkylen darstellt, optische Isomere von Verbindungen der Formel (I), Mischungen dieser optischen Isomere und Salze, insbesondere pharmazeutisch annehmbare Salze von solchen Verbindungen der Formel (I), die eine salzbildende Gruppe enthalten.

Die Erfindung betrifft in erster Linie Verbindungen der Formel (I), worin $R_1$ durch Hydroxy substituiertes Niederalkyl ist, $R_2$ Carboxyl, 1-Niederalkoxycarbonyloxyniederalkoxycarbonyl oder Niederalkanoyloxymethoxycarbonyl bedeutet, $R_3$ über ein tertiäres Stickstoffatom an den Rest -A- gebundenes Pyrrolyl, Imidazolyl, z.B. 1-Imidazolyl, 1H-Triazolyl, z.B. 1H-1,2,4-Triazol-1-yl, oder unsubstituiertes oder durch Amino, Carboxyniederalkyl, Sulfoniederalkyl oder Diniederalkylaminoniederalkyl substituiertes 1H-Tetrazolyl, z.B. 1H-Tetrazol-1-yl oder 2H-Tetrazol-2-yl, darstellt, und A durch Niederalkyl mit 1 oder 2 Kohlenstoffatomen mono- oder disubstituiertes Niederalkylen mit 1 bis 4 Kohlenstoffatomen bedeutet, optische Isomere von Verbindungen der Formel (I), z.B. das (5R,6S)-Isomere, Mischungen dieser optischen Isomere und pharmazeutisch annehmbare Salze von solchen Verbindungen der Formel (I), die eine salzbildende Gruppe aufweisen.

Die Erfindung betrifft insbesondere (5R,6S)-konfigurierte Verbindungen der Formel (I), worin $R_1$ Hydroxymethyl oder 1-Hydroxyäthyl bedeutet, $R_2$ Carboxyl ist, $R_3$ 1H-Tetrazol-1-yl oder Pyrrol-1-yl darstellt und A 1,2-Propylen ist, und pharmazeutisch annehmbare Salze davon.

Die Erfindung betrifft weiterhin die reinen optischen Isomere von solchen Verbindungen der Formel (I), welche in den Substituenten $R_1$ und/oder A weitere Chiralitätszentren besitzen, und pharmazeutisch annehmbare Salze davon.

Die Erfindung betrifft insbesondere die in den Beispielen genannten Verbindungen der Formel (I) und ihre pharmazeutisch annehmbaren Salze.

Die Verbindungen der vorliegenden Erfindung können nach an sich bekannten Verfahren hergestellt werden.

Die neuen Verbindungen werden z.B. hergestellt, indem man

a) eine Ylid-Verbindung der Formel

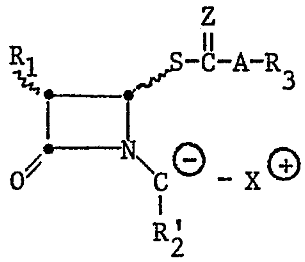

(II),

worin $R_1$, $R_3$ und A die unter Formel (I) angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

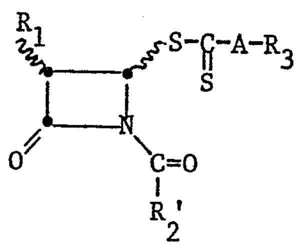

(III),

worin $R_1$, $R_3$ und A die unter Formel (I) angegebenen Bedeutungen haben, und $R_2'$ eine geschützte Carboxylgruppe darstellt, mit einer organischen Verbindung des dreiwertigen Phosphors behandelt, oder

c) eine Verbindung der Formel

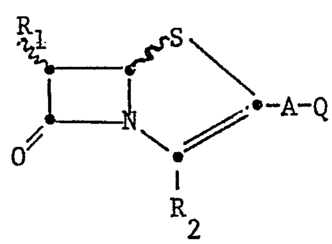

(IV),

worin $R_1$, $R_2$ und A die unter Formel (I) angegebenen Bedeutungen haben und Q eine reaktionsfähige veresterte Hydroxygruppe bedeutet, mit einem den Azaheterocyclyl-Rest $R_3$ einführenden Mittel umsetzt,

und, wenn erwünscht oder notwendig in einer erhältlichen Verbindung der Formel (I) eine geschützte Hydroxygruppe im Rest $R_1$ in die freie Hydroxygruppe überführt und/oder, wenn erwünscht, in einer erhältlichen Verbindung der Formel (I) eine geschützte Carboxylgruppe $R_2'$ in die freie, in eine unter physiologischen Bedingungen spaltbare veresterte Carboxylgruppe oder in eine andere geschützte Carboxylgruppe $R_2'$ überführt, und/oder, wenn erwünscht, eine freie Carboxylgruppe $R_2$ in eine unter physiologischen Bedingungen spaltbare veresterte Carboxylgruppe überführt, und/oder, wenn erwünscht, weitere im Rest $R_3$ enthaltene geschützte funktionelle Gruppen in die freien funktionellen Gruppen überführt, und/oder, wenn erwünscht, in einer erhältlichen Verbindung der Formel (I) einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

In den Ausgangsverbindungen der Formeln (II), (III) und (IV) sind funktionelle Gruppen, wie eine Hydroxygruppe im Rest $R_1$, sowie in den Verbindungen der Formeln (I) und (II) zusätzlich vorhandene funktionelle Gruppen im Rest $R_3$ vorzugsweise durch konventionelle Schutzgruppen, z.B. durch die obengenannten, geschützt.

a. Cyclisierung der Verbindung der Formel II

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetall-, z.B. Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. Natriumion.

Die Ylid-Verbindungen der Formel II werden in der isomeren Ylen-Form auch als Phosphoran-Verbindungen bezeichnet. In Phosphonio-Verbindungen der Formel II wird die negative Ladung durch die positiv geladene Phosphoniogruppe neutralisiert. In Phosphono-Verbindungen der Formel II wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 50°C bis etwa 100°C, erfolgen. Die Reaktion wird vorzugsweise in einem geeigneten inerten Lösungsmittel, wie einem aliphatischen, cycloaliphatischen oder aromatischen Kohlenwasserstoff, z.B. Hexan oder Benzol, einem halogenierten Kohlenwasserstoff, z.B. Methylenchlorid, einem Aether, z.B. Diäthyläther, einem cyclischen Aether, z.B. Dioxan, einem Carbonsäureamid, z.B. Dimethylformamid, einem Diniederalkylsulfoxid, z.B. Dimethylsulfoxid, oder einem Niederalkanol, z.B. Methanol, oder in einem Gemisch davon, und, falls notwendig, in einer Inertgas-, z.B. Stickstoffatmosphäre, durchgeführt.

b. Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2-N(R_b)_2$, worin $R_a$ und $R_b$ unabhängig voneinan-

der Niederalkyl, z.B. Methyl, oder Aryl, z.B. Phenyl, bedeuten. Bevorzugte Verbindungen des dreiwertigen Phosphors sind Trialkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis etwa 80°C, bevorzugt von etwa 40° bis etwa 60°C, durchgeführt, wobei man 1 Moläquivalent einer Verbindung der Formel III mit 2 Moläquivalenten der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel III in situ her und setzt es ohne Isolierung aus dem Reaktionsgemisch mit der organischen Verbindung des dreiwertigen Phosphors um, wobei die Endprodukte der Formel I entstehen.

c. Einführung des Azaheterocyclyl-Restes $R_3$

In den Verbindungen der Formel (IV) bedeutet reaktionsfähiges verestertes Hydroxy Q beispielsweise mit Halogenwasserstoffsäuren, organischen Sulfonsäuren, wie Niederalkan- oder gegebenenfalls substituierten Benzolsulfonsäuren, Niederalkancarbonsäuren oder Phosphinsäuren verestertes Hydroxy und ist in erster Linie Halogen, z.B. Chlor, Brom oder Jod, Sulfonyloxy, z.B. Methan-, Benzol-, 4-Toluol- oder 4-Brombenzolsulfonyloxy, Niederalkanoyloxy, z.B. Acetoxy, oder Phosphinoyloxy, z.B. Dimethyl- oder Diphenylphosphinoyloxy.

Ein den Azaheterocyclyl-Rest $R_3$ einführendes Mittel ist insbesondere eine Verbindung der Formel $R_3$-H, worin das an den Rest -A- zu knüpfende Ringstickstoffatom ein Wasserstoffatom trägt.

Die Umsetzung erfolgt z.B. in Gegenwart eines basischen Kondensationsmittels, beispielsweise eines Alkali- oder Erdalkalimetallhydroxids oder -carbonats, z.B. Natriumhydroxid, Natriumcarbonat, Kaliumhydroxid, Kaliumcarbonat oder Kalziumcarbonat, eines Alkalimetallniederalkanolats, z.B. Natriummethanolat, Natriumäthanolat oder Kalium-tert.-butanolat, eines aromatischen Amins, z.B. Pyridin oder Chinolin, oder eines tertiären aliphatischen Amins, wie eines Triniederalkylamins, z.B. Triäthylamin oder Diisopropyläthylamin, in einem inerten Lösungsmittel, wie einem Niederalkanol, z.B. Methanol oder tert.-Butanol, einem Amid, z.B. Dimethylformamid, oder bei Verwendung eines flüssigen Amins als basischen Kondensationsmittels in überschüssigem Amin bei Raumtemperatur oder erhöhter oder erniedrigter Temperatur, z.B. bei etwa -20° bis etwa +80°C.

Bevorzugt werden solche Ausgangsmaterialien in Formel II, III und IV verwendet, die zu den eingangs als besonders bevorzugt genannten Verbindungen der Formel I führen, insbesondere Verbindungen der Formel II oder III, die eine 3S,4R-Konfiguration aufweisen, oder der Formel IV, die eine 5R,6S-Konfiguration aufweisen.

In einer erhältlichen Verbindung der Formel I, worin eine oder mehrere funktionelle Gruppen geschützt sind, können diese, z.B. geschützte Amino-, Carboxyl-, Hydroxy- und/oder Sulfogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino, durch Umsetzen mit einer Verbindung des Palladiums, z.B. Tetrakis(triphenylphosphin)palladium, in Gegenwart von Triphenylphosphin und Behandeln mit einer Carbonsäure, z.B. 2-Aethylhexansäure, oder einem Salz davon, gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanionen liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid, Palladium oder Raney-Nickel, oder durch Behandeln mit Zink in Gegenwart einer Säure, wie Essigsäure. Eine in Form einer Phthalimidogruppe geschützte Aminogruppe kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

In einer verfahrensgemäss erhältlichen Verbindung der Formel I, worin $R_2$ eine geschützte Carboxylgruppe bedeutet und/oder worin der Rest $R_3$ geschütztes Carboxyl als Substituenten enthält, kann die Carboxylgruppe in an sich bekannter Weise freigesetzt werden. So kann man tert.-Niederalkoxycarbonyl oder in 2-Stellung durch eine trisubstituierte Silylgruppe oder in 1-Stellung durch Niederalkoxy substituiertes Niederalkoxycarbonyl oder gegebenenfalls substituiertes Diphenylmethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B.

Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzen mit einer Verbindung des Palladiums, z.B. Tetrakis(triphenylphosphin)palladium, in Gegenwart von Triphenylphosphin und unter Zusatz einer Carbonsäure, z.B. 2-Aethylhexansäure, oder einem Salz davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol, freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

In verfahrensgemäss erhältlichen Verbindungen der Formel I, worin der Rest $R_1$ durch geschütztes Hydroxyl substituiert ist und/oder worin der Rest $R_3$ geschütztes Hydroxyl als Substituenten enthält, kann die geschützte Hydroxygruppe in an sich bekannter Weise in die freie Hydroxygruppe überführt werden. Beispielsweise wird eine durch eine

geeignete Acylgruppe oder eine organische Silyl- oder Stannylgruppe geschützte Hydroxygruppe wie eine entsprechend geschützte Aminogruppe freigesetzt, eine Triniederalkylsilylgruppe z.B. auch mit Tetrabutylammoniumfluorid und Essigsäure (unter diesen Bedingungen werden durch trisubstituiertes Silyläthoxy geschützte Carboxygruppen nicht gespalten). Eine 2-Halogenniederalkylgruppe und eine gegebenenfalls substituierte Benzylgruppe werden reduktiv abgespalten.

Eine geschützte, insbesondere veresterte, Sulfogruppe wird analog einer geschützten Carboxylgruppe freigesetzt.

Andererseits können auch Verbindungen der Formel I, worin $R_2$ Carboxy, bedeutet, in Verbindungen der Formel I überführt werden, worin $R_2$ eine geschützte Carboxylgruppe, insbesondere eine veresterte Carboxylgruppe, oder eine unter physiologischen Bedingungen spaltbare veresterte Carboxylgruppe darstellt. So kann man die freie Carboxylgruppe z.B. durch Behandeln mit einer geeigneten Diazoverbindung, wie einem Diazoniederalkan, z.B. Diazomethan, oder einem Phenyldiazoniederalkan, z.B. Diphenyldiazomethan, wenn notwendig, in Gegenwart einer Lewis-Säure, wie z.B. Bortrifluorid, oder durch Umsetzen mit einem zur Verestertung geeigneten Alkohol in Gegenwart eines Veresterungsmittels, wie eines Carbodiimids, z.B. Dicyclohexylcarbodiimid, sowie Carbonyldiimidazol, verestern. Ester können auch durch Umsetzung eines gegebenenfalls in situ hergestellten Salzes der Säure mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylestern, wie Chlorameisensäureäthyl- oder Chlorameisensäureisobutylester, oder mit Halogenessigsäurehalogeniden, wie Trichloressigsäurechlorid) durch Umsetzen mit geeigneten Alkoholen, gegebenenfalls in Gegenwart einer Base, wie Pyridin, in eine veresterte Carboxylgruppe überführt

werden.
In einer Verbindung der Formel I mit einer veresterten Carboxylgruppe kann diese in eine andere veresterte Carboxylgruppe überführt werden, z.B. 2-Chloräthoxycarbonyl oder 2-Bromäthoxycarbonyl durch Behandeln mit einem Jodsalz, z.B. Natriumjodid, in 2-Jodäthoxycarbonyl. Ferner kann man in Verbindungen der Formel I, die eine in veresterter Form geschützte Carboxylgruppe $R_2'$ enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähigen Ester eines entsprechenden Alkohols in eine Verbindung der Formel I überführen, worin $R_2$ eine unter physiologischen Bedingungen spaltbare veresterte Carboxylgruppe darstellt.

In Verbindungen der Formel I kann man weiterhin einen Rest $R_3$ in einen anderen Rest $R_3$ überführen.

So kann beispielsweise in Verbindungen der Formel I, worin der Heterocyclyl-Rest $R_3$ durch eine Carboxylgruppe substituiert ist, diese Carboxylgruppe nach an sich bekannten Verfahren in eine funktionell abgewandelte Carboxylgruppe, wie in eine veresterte Carboxylgruppe oder in gegebenenfalls substituiertes Carbamoyl, überführt werden. Beispielsweise erhält man durch Umsetzen einer Verbindung der Formel I, worin $R_3$ durch Carboxyl substituiertes Heterocyclyl ist, mit einem Alkohol, insbesondere einem Niederalkanol, eine Verbindung der Formel I, worin $R_3$ durch verestertes Carboxyl, insbesondere Niederalkoxycarbonyl, substituiertes Heterocyclyl ist, wobei man vorzugsweise in Gegenwart eines geeigneten Kondensationsmittels, z.B. eines Carbodiimids,arbeitet oder das entstehende Wasser durch azeotrope Destillation entfernt. Andererseits kann man Carboxylgruppen an Resten $R_3$ auch in reaktionsfähige funktionelle Derivate, wie gemischte Anhydride, z.B. Säurehalogenide, oder aktivierte Ester, überführen und diese durch Umsetzen mit einem Alkohol, z.B. Niederalkanol, Ammoniak oder einem primären oder sekundären Amin, z.B. einem Niederalkyl- oder Diniederalkylamin, in entsprechend veresterte oder amidierte Carboxylgruppen umwandeln,

wobei man bei Verwendung von gemischten Anhydriden vorzugsweise in Gegenwart eines säurebindenden Mittels arbeitet, wie eines aromatischen oder tertiären Amins oder eines Alkalimetall- oder Erdalkalimetallcarbonats.

Weist ein Heteroaryl-Rest $R_3$ eine Hydroxygruppe auf, so lässt sich diese auf üblichem Wege veräthern. Die Umsetzung zu den entsprechenden Niederalkyl-heteroaryläthern erfolgt beispielsweise in Gegenwart von Basen, wie Alkalimetallhydroxiden oder -carbonaten, z.B. Natriumhydroxid oder Kaliumcarbonat, mit Hilfe von Diniederalkylsulfaten oder Niederalkylhalogeniden, oder mit Diazoniederalkanen, oder, in Gegenwart eines Dehydatisierungsmittels, beispielsweise Dicyclohexylcarbodiimid, mit Hilfe von Niederalkanolen. Weiterhin lässt sich Hydroxy in verestertes Hydroxy, z.B. Niederalkanoyloxy, umwandeln, beispielsweise durch Umsetzung mit dem reaktionsfähigen Derivat einer entsprechenden Niederalkancarbonsäure, z.B. Essigsäure, wie eines Anhydrids davon, z.B. des symmetrischen Anhydrids davon oder eines gemischten Anhydrids mit einer Halogenwasserstoffsäure, erforderlichenfalls in Gegenwart eines basischen Kondensationsmittels, wie eines Alkalimetallhydroxides oder -carbonates, oder einer Stickstoffbase, z.B. Pyridin. Die Umwandlung von Niederalkanoyloxy zu Hydroxy erfolgt beispielsweise durch Alkoholyse oder, vorzugsweise, Hydrolyse, z.B. durch basenkatalysierter Hydrolyse, z.B. in Gegenwart von Natriumhydroxid.

In Verbindungen der Formel I, worin $R_3$ durch Amino substituiertes Heterocyclyl bedeutet, kann die Aminogruppe in eine substituierte Aminogruppe, wie eine Niederalkylamino-, Diniederalkylamino-, Niederalkylenamino- oder Niederalkanoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali- oder Erdalkalimetalls oder einer heteroaromatischen Stickstoffbase, z.B. Pyridin. In analoger Weise kann Amino durch Behandeln mit einem Niederalkylendihalogenid oder -disulfonat in Niederalkylenamino und durch Behandeln mit dem reaktionsfähigen funktionellen Derivat einer Niederalkancarbonsäure, z.B. dem entsprechenden Carbonsäurehalogenid, in Niederalkanoylamino umgewandelt werden.

Salze von Verbindungen der Formel I mit salzbildenden Gruppen können in an sich bekannter Weise hergestellt werden. So kann man Salze von Verbindungen der Formel I mit einer freien Carboxylgruppe z.B. durch Behandeln mit Metallverbindungen, wie Alkalimetallsalzen von geeigneten organischen Carbonsäuren, z.B. dem Natriumsalz der α-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von Isomeren können nach an sich bekannten Methoden in die einzelnen Isomeren aufgetrennt werden, Gemische von diastereomeren Isomeren z.B. durch fraktioniertes Kristallisieren, Adsorptionschromatographie (Kolonnen- oder Dünnschichtchromatographie) oder andere geeignete Trennverfahren.

Die Spaltung von erhaltenen Racematen in ihre optischen Antipoden kann auf verschiedenen Wegen erfolgen.

Einer dieser Wege besteht darin, dass man ein Racemat mit einem optisch aktiven Hilfsstoff reagieren lässt, das dabei entstandene Gemisch zweier diastereomerer Verbindungen mit Hilfe von geeigneten physikalisch-chemischen Methoden trennt und die einzelnen diastereomeren Verbindungen dann in die optisch aktiven Verbindungen spaltet.

Zur Trennung in Antipoden besonders geeignete Racemate sind solche, die eine saure Gruppe besitzen, wie z.B. Racemate von Verbindungen der Formel I, worin $R_2$ Carboxy ist. Diese sauren Racemate können mit optisch aktiven Basen, z.B. Estern von optisch aktiven Aminosäuren, oder (-)-Brucin, (+)-Chinidin, (-)-Chinin, (+)-Cinchonin, (+)-Dehydroabietylamin, (+)- und (-)-Ephedrin, (+)- und (-)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol, (+)- oder (-)-2-Octanol verestert sein oder werden, worauf nach erfolgter Isolierung des gewünschten Diastereomeren die Carboxylgruppe freigesetzt wird.

Zur Racemattrennung kann auch die Hydroxygruppe mit optisch aktiven Säuren oder deren reaktionsfähigen, funktionellen Derivaten verestert

werden, wobei sich diastereomere Ester bilden. Solche Säuren sind beispielsweise (-)-Abietinsäure, D(+)- und L(-)-Aepfelsäure, N-acylierte optisch aktive Aminosäure, (+)- und (-)-Camphansäure, (+)- und (-)-Ketopinsäure, L(+)-Ascorbinsäure, (+)-Camphersäure, (+)-Campher-10-sulfonsäure(β), (+)- oder (-)-α-Bromcampher-π-sulfonsäure, D(-)-Chinasäure, D(-)-Isoascorbinsäure, D(-)- und L(+)-Mandelsäure, (+)-1-Menthoxyessigsäure, D(-)- und L(+)-Weinsäure und deren Di-O-benzoyl- und Di-O-p-toluylderivate.

Durch Umsetzung mit optisch aktiven Isocyanaten, wie mit (+)- oder (-)-1-Phenyläthylisocyanat, können Verbindungen der Formel (I), worin $R_2$ geschütztes Carboxy und der Rest $R_1$ durch Hydroxy substituiertes Niederalkyl bedeutet, in ein Gemisch diastereomerer Urethane umgewandelt werden.

Basische Racemate, z.B. Verbindungen der Formel I, worin der Rest $R_3$ durch Amino substituiert ist, können mit den genannten optischen aktiven Säure diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch aktiven Absorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden gegenüber biologischem Material wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produktes aus.

Die Auftrennung von Diastereomeren in die einzelnen Racemate und der Racemate in die optischen Antipoden, kann auf einer beliebigen Verfahrensstufe, d.h. z.B. auch auf der Stufe der Ausgangsverbindungen der Formeln II bis IV oder auf einer beliebigen Stufe des nachstehend beschriebenen Verfahrens zur Herstellung des Ausgangsmaterials der Formel II oder IV, durchgeführt werden.

Bei allen nachträglichen Umwandlungen erhaltener Verbindungen der Formel I werden solche Reaktionen bevorzugt, die unter neutralen oder alkalischen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, gebildet werden. Beispielsweise kann ein Ausgangsmaterial der Formel II, worin Z Sauerstoff ist, aus einer Verbindung der Formel II, worin Z eine, wie nachstehend beschrieben, gegebenenfalls substituierte Methylidengruppe ist, durch Ozonisierung und anschliessende Reduktion des gebildeten Ozonids, analog dem weiter unten angegebenen Verfahren (Stufe 2.3), in situ hergestellt werden, worauf in der Reaktionslösung die Cyclisierung zur Verbindung der Formel I erfolgt.

Die Ausgangsverbindungen der Formeln II, III und IV und die Vorstufen können, wie in den Reaktionsschemata I und II angegeben, hergestellt werden:

Reaktionsschema I

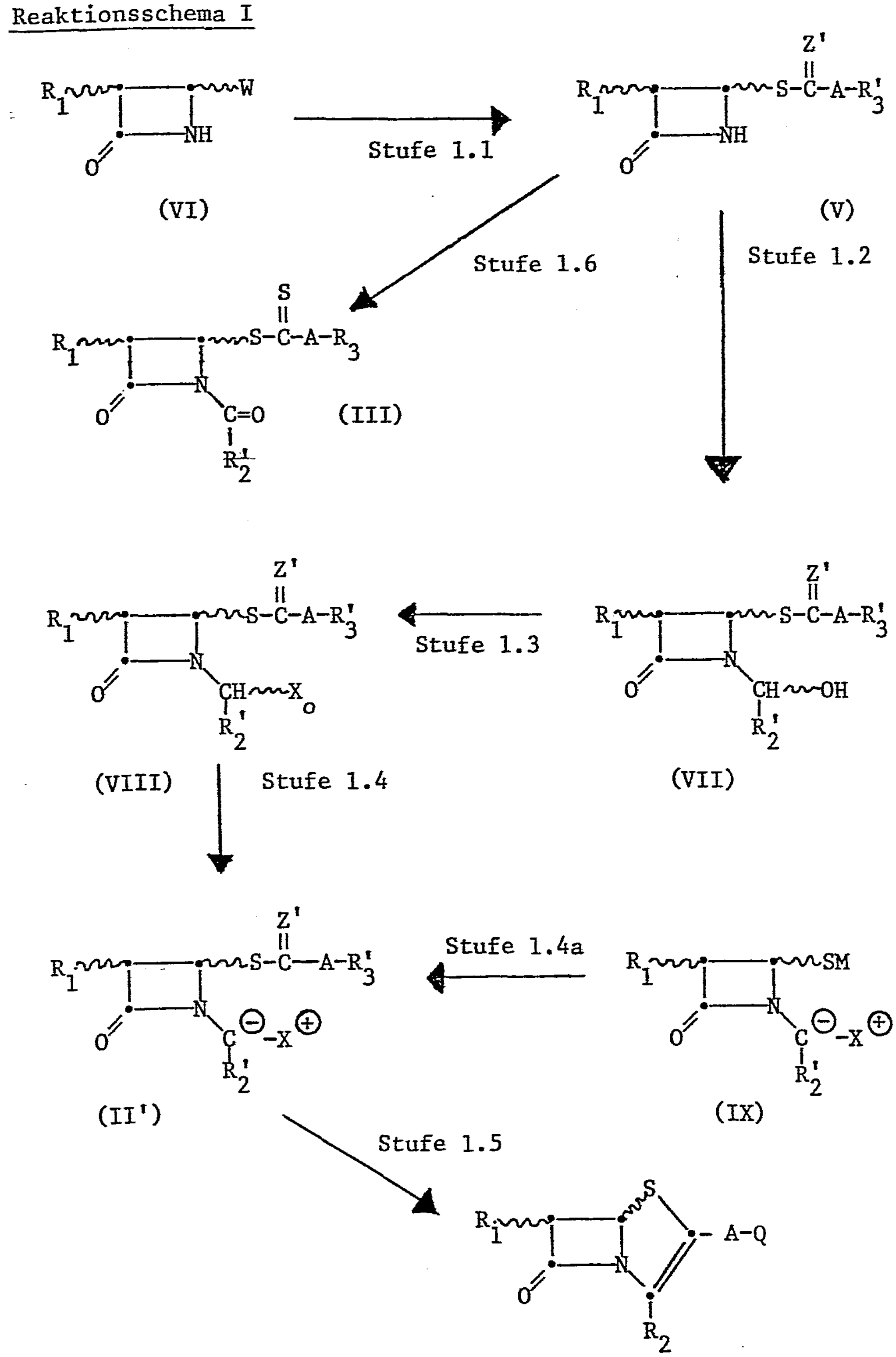


(VI) (V) (III) (VIII) (VII) (II') (IX)

(IV)

In den Verbindungen der Formeln V, VII, VIII und II' ist Z' Sauerstoff, Schwefel oder auch eine gegebenenfalls durch einen oder zwei Substituenten Y substituierte Methylidengruppe, die durch Oxidation in eine Oxogruppe Z überführt werden kann. Ein Substituent Y dieser Methylidengruppe ist ein organischer Rest, beispielsweise gegebenenfalls substituiertes Niederalkyl, z.B. Methyl oder Aethyl, Cycloalkyl, z.B. Cyclopentyl oder Cyclohexyl, Phenyl oder Phenylniederalkyl, z.B. Benzyl, oder insbesondere eine, inklusive mit einem optisch aktiven Alkohol, wie 1-Menthol, veresterte Carboxylgruppe, z.B. einer der unter $R_2$ erwähnten gegebenenfalls substituierten Niederalkoxycarbonyl- oder Arylmethoxycarbonylreste oder auch 1-Menthyloxycarbonyl. Die Methylidengruppe Z' trägt bevorzugt einen der genannten Substituenten. Hervorzuheben sind die Methoxycarbonylmethyliden-, Aethoxycarbonylmethylidenund die 1-Menthyloxycarbonylmethylidengruppe Z'. Letztere kann zur Herstellung optisch aktiver Verbindungen der Formeln V, VII, VIII und II' verwendet werden.

In den Verbindungen der Formeln V, VII, VIII und II' steht $R_3'$ entweder für den Rest $R_3$ oder für eine reaktionsfähige veresterte Hydroxygruppe Q.

In den Verbindungen der Formeln V bis IX und II' enthält der Rest $R_1$ bevorzugt eine der genannten geschützten Hydroxygruppen, z.B. gegebenenfalls substituiertes 1-Phenylniederalkoxy, gegebenenfalls substituiertes Phenylniederalkoxycarbonyloxy, oder trisubstituiertes Silyloxy.

Stufe 1.1: Ein Thio-azetidinon der Formel V wird erhalten, indem man ein 4-W-Azetidinon der Formel VI, worin W eine nucleofuge Abgangsgruppe bedeutet, mit einer Mercaptoverbindung der Formel

$$R_3'-A-C(=Z')-SH$$

oder einem Salz, z.B. einem Alkalimetall-, wie Natrium- oder Kaliumsalz davon, behandelt, und, wenn erwünscht, in einer erhältlichen Verbindung der Formel V, worin der Rest $R_1$ durch Hydroxy substituiert ist, Hydroxy in geschütztes Hydroxy überführt.

Die nucleofuge Abgangsgruppe W in einem Ausgangsmaterial der Formel VI ist ein durch den nucleophilen Rest

$R_3'$-A-C(=Z')-S-

ersetzbarer Rest. Solche Gruppen W sind beispielsweise Acyloxyreste, Sulfonylreste $R_o-SO_2-$, worin $R_o$ ein organischer Rest ist, Azido oder Halogen. In einem Acyloxyrest W ist Acyl z.B. der Rest einer organischen Carbonsäure, inklusive einer optisch aktiven Carbonsäure, und bedeutet beispielsweise Niederalkanoyl, z.B. Acetyl oder Propionyl, gegebenenfalls substituiertes Benzoyl, z.B. Benzoyl oder 2,4-Dinitrobenzoyl, Phenylniederalkanoyl, z.B. Phenylacetyl, oder den Acylrest einer der oben genannten optisch aktiven Säuren. In einem Sulfonylrest $R_o-SO_2-$ ist $R_o$ beispielsweise gegebenenfalls durch Hydroxy substituiertes Niederalkyl, wie Methyl, Aethyl oder 2-Hydroxyäthyl, ferner auch entsprechendes substituiertes optisch aktives Niederalkyl, z.B. (2R)- oder (2S)-1-Hydroxyprop-2-yl, durch einen optisch aktiven Rest substituiertes Methyl, wie Campheryl, oder Benzyl, oder gegebenenfalls substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl- oder 2-Hydroxyäthylsulfonyl, Acetoxy oder Chlor.

Die nucleophile Substitution kann unter neutralen oder schwach basischen Bedingungen in Gegenwart von Wasser und gegebenenfalls einem mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingesetellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, Acetonitril und ähnliche verwendet werden. Die Reaktion wird üblicherweise bei Raumtemperatur

durchgeführt, kann aber auch bei erhöhter oder erniedrigter Temperatur durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

In die Reaktion können sowohl optisch inaktive cis- oder trans-Verbindungen der Formel VI, als auch Mischungen davon, oder entsprechende optisch aktive Verbindungen eingesetzt werden. Die eintretende Gruppe $R_3'$-A-C(=Z')-S- wird von der Gruppe $R_1$ bevorzugt in die trans-Stellung dirigiert, unabhängig davon, ob W zur Gruppe $R_1$ in cis- oder trans-Stellung steht. Obwohl die trans-Isomeren überwiegend gebildet werden, können doch gelegentlich auch die cis-Isomeren isoliert werden. Die Auftrennung der cis- und trans-Isomeren erfolgt wie oben beschrieben, nach konventionellen Methoden, insbesondere durch Chromatographie und/oder Kristallisation.

Die nachträgliche Ozonisierung einer Methyliden-Gruppe Z' kann wie weiter unten angegeben durchgeführt werden. Ein erhaltenes Racemat der Formel V kann in die optisch aktiven Verbindungen getrennt werden.

Ein Azetidinon der Formel VI, worin $R_1$ Acetoxymethyl bedeutet, ist in der Deutschen Offenlegungsschrift Nr. 29 50 898 beschrieben.

Andere Azetidinone der Formel VI können nach an sich bekannten Verfahren hergestellt werden, beispielsweise indem man einen Vinylester der Formel $R_1$-CH=CH-W mit Chlorsulfonylisocyanat umsetzt und das entstandene Cycloaddukt mit einem Reduktionsmittel, z.B. Natriumsulfit, umsetzt. Bei dieser Synthese werden gewöhnlich Mischungen von cis- und trans-Isomeren erhalten, die gewünschtenfalls in die reinen cis- oder trans-Isomeren, z.B. durch Chromatographie und/oder Kristallisation oder Destillation, aufgetrennt werden können. Die reinen cis- und trans-Isomeren liegen als Racemate vor und können in ihre optischen Antipoden getrennt werden, beispielsweise wenn Acyl in einem Acyloxyrest W in Verbindungen der Formel VI von einer optisch aktiven Säure

stammt. Die Verbindungen der Formel VI, insbesondere ihre optisch aktiven Vertreter, können auch nach dem unter dem Reaktionsschema II angegebenen Verfahren hergestellt werden.

Stufe 1.2: Eine α-Hydroxycarbonsäureverbindung der Formel VIII wird erhalten, indem man eine Verbindung der Formel V mit einer Glyoxylsäure-Verbindung der Formel $OHC-R_2'$ oder einem geeigneten Derivat davon, wie einem Hydrat, Hemihydrat oder Halbacetal, z.B. einem Halbacetal mit einem Niederalkanol, z.B. Methanol oder Aethanol, umsetzt und, wenn gewünscht, in einer erhältlichen Verbindung der Formel VII, worin der Rest $R_1$ durch Hydroxy substituiert ist, Hydroxy in geschütztes Hydroxy überführt.

Die Verbindung VII erhält man üblicherweise als Gemisch der beiden Isomere (bezüglich der Gruppierung $>CH{\sim}OH$). Man kann aber auch die reinen Isomere davon isolieren.

Die Anlagerung der Glyoxylsäureesterverbindung an das Stickstoffatom des Lactamrings findet bei Raumtemperatur oder, falls notwendig, unter Erwärmen, z.B. bis etwa 100°C, und zwar in Abwesenheit eines eigentlichen Kondensationsmittels und/oder ohne Bildung eines Salzes statt. Bei Verwendung des Hydrats der Glyoxylsäureverbindung wird Wasser gebildet, das, wenn notwendig, durch Destillation, z.B. azeotrop, oder durch Verwendung eines geeigneten Dehydrationsmittels, wie eines Molekularsiebs, entfernt wird. Vorzugsweise arbeitet man in Gegenwart eines geeigneten Lösungsmittels, wie z.B. Dioxan, Toluol oder Dimethylformamid, oder Lösungsmittelgemisches, wenn erwünscht oder notwendig, in der Atmosphäre eines Inertgases, wie Stickstoff.

In die Reaktion können sowohl reine optisch inaktive cis- oder trans-Verbindungen der Formel V, als auch Mischungen davon, oder entsprechende optisch aktive Verbindungen eingesetzt werden. Ein erhaltenes Racemat der Formel VII kann in die optisch aktiven Verbindungen getrennt werden.

Stufe 1.3: Verbindungen der Formel VIII, worin $X_o$ für eine reaktionsfähige veresterte Hydroxygruppe, insbesondere für Halogen oder organisches Sulfonyloxy steht, werden hergestellt, indem man in einer Verbindung der Formel VII die sekundäre Hydroxygruppe in eine reaktionsfähige veresterte Hydroxygruppe, insbesondere in Halogen, z.B. Chlor oder Brom, oder in eine organische Sulfonyloxygruppe, wie Niederalkansulfonyloxy, z.B. Methansulfonyloxy, oder Arensulfonyloxy, z.B. Benzol- oder 4-Methylbenzolsulfonyloxy, umwandelt.

In den Ausgangsverbindungen der Formel VII steht $R_1$ vorzugsweise für durch eine geschützte Hydroxygruppe substituiertes Niederalkyl.

Die Verbindungen der Formel VIII kann man in Form von Gemischen der Isomere (bezüglich der Gruppierung $>CH{\sim}X_o$) oder in Form von reinen Isomere erhalten.

Die obige Reaktion wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphoniumhalogenid, wie Triphenylphosphondibromid oder -dijodid, oder einem geeigneten organischen Sulfonsäurehalogenid, wie -chlorid, vorzugsweise in Gegenwart eines basischen, in erster Linie eines organischen basischen Mittels, wie eines aliphatischen tertiären Amins, z.B. Triäthylamin, Diisopropyläthylamin oder "Polystyrol-Hünigbase", oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, wenn notwendig, unter Kühlen und/oder in der Atmosphäre eines Inertgases, wie Stickstoff.

In einer so erhältlichen Verbindung der Formel VIII kann eine reaktionsfähige veresterte Hydroxygruppe $X_o$ in an sich bekannter Weise in eine andere reaktionsfähige veresterte Hydroxygruppe umgewandelt werden.

So kann man z.B. ein Chloratom durch Behandeln der entsprechenden Chlorverbindung mit einem geeigneten Bromid- oder Jodidsalz, wie Lithiumbromid oder -jodid, vorzugsweise in Gegenwart eines geeigneten Lösungsmittels, wie Aether, durch ein Brom- bzw. Jodatom austauschen.

In die Reaktion können sowohl reine optische inaktive cis- oder trans-Verbindungen der Formel VII als auch Mischungen davon, oder entsprechende optisch aktive Verbindungen eingesetzt werden. Ein erhaltenes Racemat der Formel VIII kann in die optisch aktiven Verbindungen getrennt werden.

Stufe 1.4: Das Ausgangsmaterial der Formel II' wird erhalten, indem man eine Verbindung der Formel VIII, worin $X_o$ für eine reaktionsfähige veresterte Hydroxygruppe steht, mit einer geeigneten Phosphin-Verbindung, wie einem Triniederalkylphosphin, z.B. Tri-n-butyl-phosphin, oder einem Triaryl-phosphin, z.B. Triphenyl-phosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt.

Die obige Reaktion wird vorzugsweise in Gegenwart eines geeigneten inerten Lösungsmittels, wie eines Kohlenwasserstoffs, z.B. Hexan, Cyclohexan, Benzol, Toluol oder Xylol, oder eines Aethers, z.B. Dioxan, Tetrahydrofuran oder Diäthylenglykol-dimethyläther, oder eines Lösungsmittelgemisches vorgenommen. Je nach Reaktionsfähigkeit arbeitet man unter Kühlen oder bei erhöhter Temperatur, etwa zwischen -10° und +100°C, bevorzugt bei etwa 20° bis 80°C, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man bei der Verwendung einer Phosphinverbindung üblicherweise in Gegenwart eines basischen Mittels, wie einer organischen Base, z.B. eines Amins, wie Triäthylamin, Diisopropyläthylamin oder

"Polystyrol-Hünigbase", und gelangt so direkt zum Ylid-Ausgangsmaterial der Formel II (bzw. II'), das aus dem entsprechenden Phosphoniumsalz gebildet wird.

Eine Ausgangsverbindung der Formel II, worin $X^{\oplus}$ eine Phosphonogruppe zusammen mit einem Kation ist, wird vorzugsweise in situ hergestellt, indem man eine erhältliche Verbindung der Formel

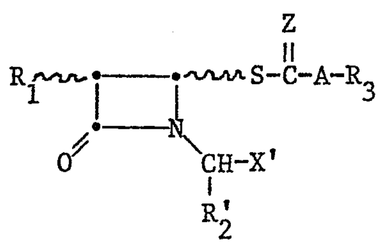

(IIa),

worin X' eine Phosphonogruppe bedeutet, mit einem geeigneten basischen Reagenz, wie einer anorganischen Base, z.B. einem Alkalimetallcarbonat, wie Natrium- oder Kaliumcarbonat, oder einer organischen Base, wie einem Triniederalkylamin, z.B. Triäthylamin, oder einer cyclischen Base vom Amidintyp, wie einer entsprechenden Diazabicycloalkenverbindung, z.B. 1,5-Diazabicyclo[5.4.0]undec-5-en, behandelt.

In die Reaktion können sowohl reine, optisch inaktive cis- oder trans-Verbindungen der Formel VIII, als auch Mischungen davon, oder entsprechende optisch aktive Verbindungen eingesetzt werden. Ein erhaltenes Racemat der Formel II' kann in die optisch aktiven Verbindungen getrennt werden.

Stufe 1.4a

Eine Ausgangsverbindung der Formel II', worin Z' für Oxo steht, kann weiterhin erhalten werden, indem man ein Mercaptid der Formel IX, worin M für ein Metallkation steht, mit einem den Rest $R_3'$-A-C(=O)- einführenden Acylierungsmittel behandelt.

In dem Ausgangsmaterial der Formel IX ist das Metallkation M beispielsweise ein Kation der Formel $M^+$ oder $M^{2+}/2$, wobei $M^+$ insbesondere für ein Silberkation und $M^{2+}$ insbesondere für das zwei-

wertige Kation eines geeigneten Uebergangsmetalls, z.B. Kupfer, Blei oder Quecksilber, steht.

Ein den Rest $R_3'$-A-C(=O)- einführendes Acylierungsmittel ist z.B. die Säure $R_3'$-A-COOH oder ein reaktionsfähiges funktionelles Derivat davon, wie ein Säurehalogenid, z.B. Chlorid oder Bromid, Azid oder Anhydrid davon.

Die Acylierung erfolgt, wenn die freie Säure der Formel $R_3'$-A-COOH eingesetzt wird, z.B. in Gegenwart eines geeigneten wasserentziehenden Mittels, wie eines Carbodiimids, z.B. N,N'-Dicyclohexylcarbodiimid, oder, wenn ein Säurederivat eingesetzt wird, in Gegenwart eines geeigneten säurebindenden Mittels, wie einer tertiären aliphatischen oder aromatischen Base, z.B. Triäthylamin, Pyridin oder Chinolin, in einem inerten Lösungsmittel, wie einem chlorierten Kohlenwasserstoff, z.B. Methylenchlorid, oder einem Aether, z.B. Diäthyläther oder Dioxan, bei Raumtemperatur oder unter Erhitzen oder Kühlen, z.B. in einem Temperaturbereich von ca. -50° bis ca. +60°C, insbesondere bei ca. -30° bis ca. +20°C.

Die Ausgangsverbindungen der Formel IX können beispielsweise hergestellt werden, indem man ein Azetidinon der Formel

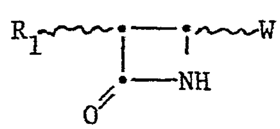

(VI)

durch Umsetzen mit einem Alkalimetallsalz, z.B. dem Natriumsalz, einer Thioniederalkancarbonsäure, z.B. Thioessigsäure, oder des Triphenylmethylmercaptans in eine Verbindung der Formel

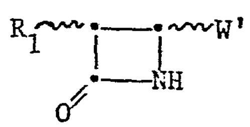

(VI')

überführt, worin W' Triphenylmethylthio oder Niederalkanoylthio, z.B. Acetylthio, bedeutet, diese analog dem in den Reaktionsstufen 1.2, 1.3 und 1.4 beschriebenen Verfahren, in eine Verbindung der Formel

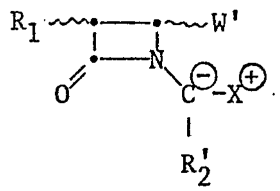

(X)

überführt und diese in Gegenwart einer Base, z.B. Pyridin oder Tri-n-butylamin, in einem geeigneten Lösungsmittel, z.B. Diäthyläther oder Methanol, mit einem Salz der Formel MA worin M die obige Bedeutung hat, insbesondere aber für ein Silber-Kation steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt.

Verbindungen der Formel (II'), worin $R_3'$ für eine reaktionsfähige veresterte Hydroxygruppe steht, können durch Umsetzen mit einem den Azaheterocyclyl-Rest $R_3$ einführenden Mittel in Verbindungen der Formel (II') überführt werden, worin $R_3'$ für den Rest $R_3$ steht, wobei beispielsweise die in Verfahren c) angegebenen Reaktionsbedingungen angewendet werden.

Die Ylide der Formel II', worin Z' Sauerstoff oder Schwefel ist, können direkt in die Cyclisierungsreaktion zur Herstellung der Endprodukte der Formel I eingesetzt werden. Man kann aber auch in Verbindungen der Formel II', worin $R_1$ eine geschützte Hydroxygruppe, z.B. eine hydrolytisch leicht spaltbare geschützte Hydroxygruppe, wie trisubstituiertes Silyloxy als Substituenten enthält, zuerst die Hydroxyschutzgruppe abspalten und dann die erhaltene Verbindung der Formel II', worin $R_1$ durch Hydroxy substituiertes Niederalkyl ist, in die Cyclisierungsreaktion einsetzen.

In den Verbindungen II', V,VII und VIII kann eine gegebenenfalls substituierte Methylidengruppe Z' durch Ozonisierung und anschliessende Reduktion des gebildeten Ozonids, gemäss dem nachfolgend in der Stufe 2.3 beschriebenen Verfahren, in die Oxogruppe Z überführt werden.

Stufe 1.5

Die Ausgangsverbindung der Formel (IV) wird erhalten, indem man ein Ylid der Formel (II'), worin Z' Sauerstoff oder Schwefel bedeutet und $R_3'$ für eine reaktionsfähige veresterte Hydroxygruppe Q steht, ringschliesst, und gegebenenfalls eine geschützte Carboxylgruppe $R_2'$ in einer erhältlichen Verbindung in die freie Carboxylgruppe $R_2$ überführt.

Der Ringschluss kann beispielsweise so durchgeführt werden, wie es bei der Herstellung von Verbindungen der Formel (I) aus den Yliden der Formel (II) beschrieben ist (Verfahren a.).

Die Ueberführung einer geschützten Carboxylgruppe $R_2'$ in eine freie Carboxylgruppe $R_2$ in einer erhältlichen Verbindung der Formel IV kann in analoger Weise durchgeführt werden, wie oben bei den Verbindungen der Formel I beschrieben.

Stufe 1.6

Eine Verbindung der Formel (III) wird erhalten, indem man ein Azetidinon der Formel (V), worin Z' Schwefel bedeutet, mit einer Verbindung der Formel $R_2'$-COOH oder insbesondere einem reaktionsfähigen Derivat, wie einem Säurehalogenid, z.B. dem Säurechlorid, davon bei einer Temperatur von 20° bis 80°C, bevorzugt bei 40° bis 60°C, in einem inerten Lösungsmittel, wie einem der bei der Umsetzung von Verbindungen der Formel III zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, z.B. Triäthylamin, eines aromatischen Amins, z.B. Pyridin, oder insbesondere eines Alkalimetall- oder Erdalkalimetallcarbonats oder -hydrogencarbonats, z.B. Kaliumcarbonat oder Calciumcarbonat.

Ausgangsverbindungen der Formel VI, worin W ein Sulfonylrest der Formel $R_o$-$SO_2$- ist, können auch nach dem folgenden Reaktionsschema II hergestellt werden.

Reaktionsschema II

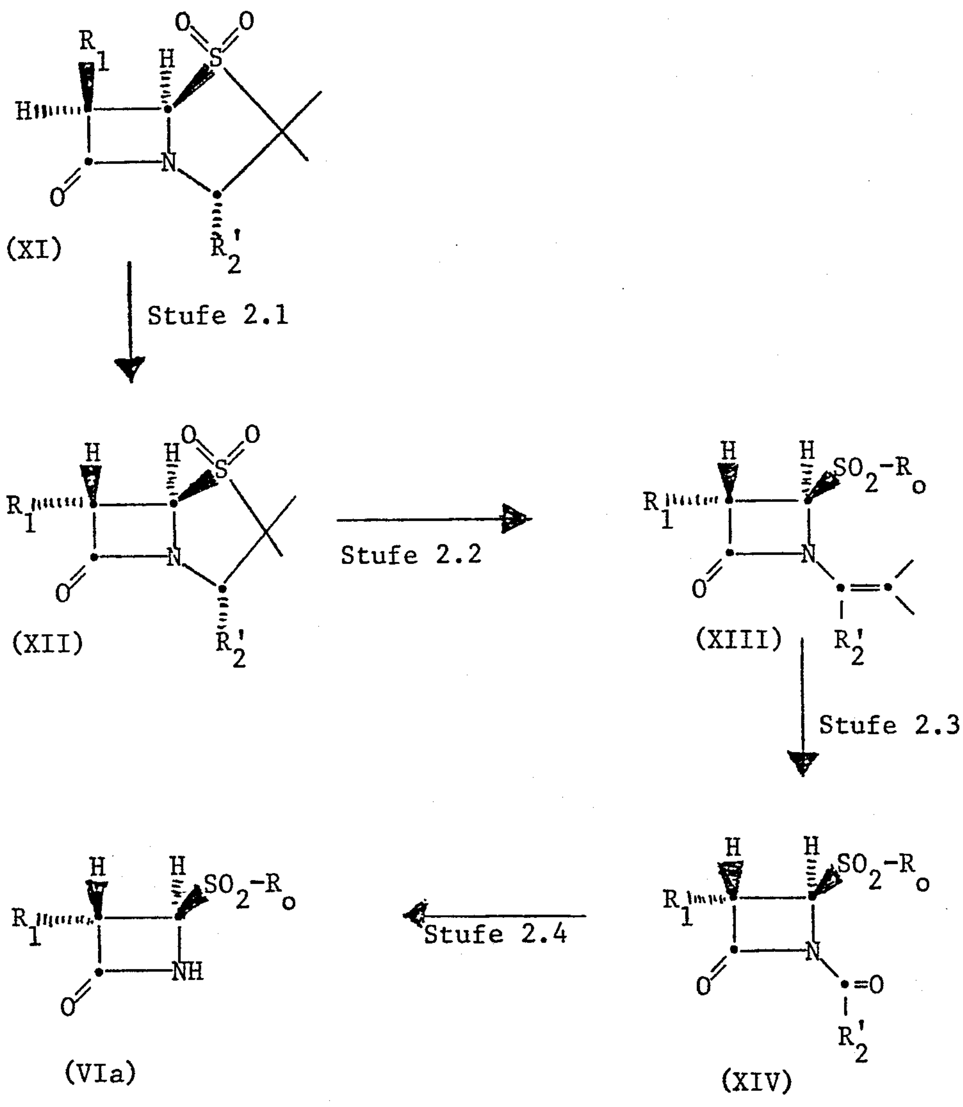


(XI) (XII) (XIII) (XIV) (VIa)

In den Verbindungen der Formeln (XI) bis (XIV) und (VIa) steht $R_1$ für durch Hydroxy oder insbesondere für durch eine geschützte Hydroxygruppe substituiertes Niederalkyl.

Stufe 2.1

Verbindungen der Formel (XII) können hergestellt werden, indem man eine Verbindung der Formel (XI) epimerisiert.

Die Epimerisierung erfolgt beispielsweise in Gegenwart eines basischen Mittels, wie eines Amins, z.B. eines Triniederalkylamins, z.B. Triäthylamin oder Aethyl-diisopropylamin, eines tertiären Amins, z.B. N,N-Dimethylanilin, eines aromatischen Amins, z.B. Pyridin, oder eines bicyclischen Amins, z.B. 1,5-Diazabicyclo[5,4,0]undec-5-en oder 1,5-Diazabicyclo[4,3,0]non-5-en, oder eines Alkalimetallniederalkanolats, z.B. Natriummethanolat, Natriumäthanolat oder Kalium-tert.-butanolat, in einem inerten Lösungsmittel, beispielsweise einem Aether, z.B. Diäthyläther, Dimethoxyäthan, Tetrahydrofuran oder Dioxan, Acetonitril oder Dimethylformamid, gegebenenfalls bei etwas erhöhter oder erniedrigter Temperatur, z.B. bei 0°C bis 50°C, vorzugsweise bei Raumtemperatur.

In den verfahrensgemäss erhältlichen Verbindungen der Formel (XII) kann eine im Rest $R_1$ enthaltende geschützte Hydroxygruppe durch eine andere geschützte Hydroxygruppe, beispielsweise eine hydrogenolytisch spaltbare geschützte Hydroxygruppe durch eine solvolytisch spaltbare geschützte Hydroxygruppe, ersetzt werden. Hydroxyschutzgruppen sind insbesondere die oben genannten; hydrogenolytisch abspaltbare Schutzgruppen beispielsweise wie angegeben substituiertes 1-Phenylniederalkyl oder Phenylniederalkoxycarbonyl, solvolytisch abspaltbare Schutzgruppen beispielsweise wie angegeben trisubstituiertes Silyl.

Die Umsetzung kann so durchgeführt werden, dass man zunächst die hydrogenolytisch abspaltbare Hydroxyschutzgruppe entfernt und in die entstandene Verbindung der Formel XII, worin $R_1$ durch Hydroxy substituiertes Niederalkyl ist, eine solvolytisch abspaltbare Hydroxyschutzgruppe einführt.

Die Abspaltung der hydrogenolytisch abspaltbaren Schutzgruppe erfolgt z.B. mit Wasserstoff oder einem Wasserstoffdonator, z.B. Cyclohexen oder Cyclohexadien, in Gegenwart eines Hydrierungskatalysators, wie eines Palladiumkatalysators, z.B. Palladium auf Kohle, in einem inerten Lösungsmittel, wie einem halogenierten Kohlenwasserstoff, z.B. Methylenchlorid, einem Niederalkanol, z.B. Methanol oder Aethanol, einem Aether, z.B. Dioxan oder Tetrahydrofuran, oder auch in Wasser oder in Gemischen davon, bei einer Temperatur von etwa 0° bis etwa 80°C, vorzugsweise bei Raumtemperatur. Die Abspaltung kann auch mit einem reduzierenden Metall, wie Zink, oder einer reduzierenden Metalllegierung, z.B. Kupfer-Zink-Legierung, in Gegenwart eines Protonenabgebenden Mittels, wie einer organischen Säure, z.B. Essigsäure, oder auch eines Niederalkanols, z.B. Aethanol, durchgeführt werden.

Die Einführung der solvolytisch abspaltbaren Hydroxyschutzgruppe erfolgt beispielsweise mit einer Verbindung der Formel $R'-X_3$, worin R' die Hydroxyschutzgruppe und $X_3$ z.B. eine reaktionsfähige veresterte Hydroxygruppe, beispielsweise Halogen, z.B. Chlor, Brom oder Jod, oder Sulfonyloxy, wie Methansulfonyloxy, Benzolsulfonyloxy oder 4-Toluolsulfonyloxy, bedeutet.

Ausgangsverbindungen der Formel (XI) sind beispielsweise aus der Deutschen Offenlegungsschrift 3 039 504 und aus der britischen Patentanmeldung 20 61 930 bekannt.

<u>Stufe 2.2</u>

Eine Verbindung der Formel (XIII) kann hergestellt werden, indem man eine Penam-Verbindung der Formel (XII) mit einem basischen Mittel und mit einem den Rest $R_o$ einführenden Veresterungsmittel behandelt.

Ein geeignetes basisches Mittel ist beispielsweise eines der unter Stufe 2.1 genannten basischen Mittel, insbesondere eines der genannten bicyclischen Amine, ferner auch ein Alkalimetallamid oder -hydrid, z.B. Natriumamid oder Natriumhydrid.

Ein Rest $R_o$ ist beispielsweise einer der unter Stufe 1.1 genannten organischen Reste, insbesondere gegebenenfalls substituiertes Niederalkyl, z.B. Methyl, Aethyl oder 2-Hydroxyäthyl, oder Benzyl.

Ein den Rest $R_o$ einführendes Veresterungsmittel ist z.B. eine Verbindung der Formel $R_o$-$X_4$, worin $X_4$ reaktionsfähiges verestertes Hydroxy, z.B. Halogen, wie Chlor, Brom oder Jod, oder Sulfonyloxy, wie Methansulfonyloxy, Benzolsulfonyloxy oder 4-Toluolsulfonyloxy, bedeutet. Zur Einführung eines 2-Hydroxyäthylrestes ist auch Aethylenoxid geeignet.

Die Umsetzung wird vorzugsweise zweistufig durchgeführt, wobei man in der ersten Stufe die Penam-Verbindung der Formel (XII) mit mindestens äquimolekularen Mengen des basischen Mittels behandelt und ein erhältliches Zwischenprodukt der Formel

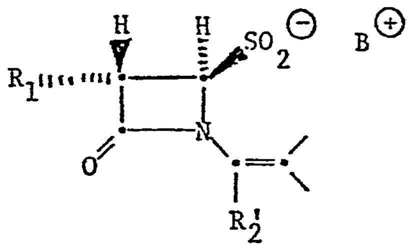

(XIIa)

worin $B^{\oplus}$ die protonierte Form (Kation) des basischen Mittels darstellt, vorzugsweise ohne Isolierung aus dem Reaktionsgemisch mit dem Veresterungsmittel umsetzt. Die Reaktion wird in einem inerten Lösungsmittel, beispielsweise einem Aether, z.B. Diäthyläther, Dimethoxyäthan, Tetrahydrofuran oder Dioxan, in Acetonitril, Dimethylformamid oder Hexamethylphosphorsäuretriamid, gegebenenfalls bei etwas erhöhter oder erniedrigter Temperatur, z.B. bei etwa 0°C bis 50°C, vorzugsweise bei Raumtemperatur, durchgeführt. In einer bevorzugten Ausführungsform des Verfahrens wird die Penam-Verbindung der Formel (XII) in situ hergestellt, indem man wie unter Stufe 2.1 beschrieben, eine Verbindung der Formel (XI) zuerst mit katalytischen Mengen des basischen Mittels, z.B. 1,5-Diazabicyclo[5,4,0]undec-5-en, behandelt, und dann mit zumindest äquimolaren Mengen des gleichen basischen

Mittels und des Veresterungsmittels weiter zu den Verbindungen der Formel (XIII) umsetzt.

Stufe 2.3

Ein Oxalyl-azetidinon der Formel (XIV) kann hergestellt werden, indem man eine Verbindung der Formel (XIII) ozonisiert und das gebildete Ozonid reduktiv zur Oxo-Verbindung spaltet.

Die Ozonisierung wird üblicherweise mit einem Ozon-Sauerstoff-Gemisch in einem inerten Lösungsmittel, wie einem Niederalkanol, z.B. Methanol oder Aethanol, einem Niederalkanon, z.B. Aceton, einem gegebenenfalls halogenierten Kohlenwasserstoff, z.B. einem Halogenniederalkan, wie Methylenchlorid oder Tetrachlorkohlenstoff, oder in einem Lösungsmittelgemisch, inkl. einem wässrigen Gemisch, vorzugsweise unter Kühlen, z.B. bei Temperaturen von etwa -80° bis etwa 0°C, durchgeführt.

Ein als Zwischenprodukt erhaltenes Ozonid wird, üblicherweise ohne isoliert zu werden, reduktiv zu einer Verbindung der Formel XIV gespalten, wobei man katalytisch aktivierten Wasserstoff, z.B. Wasserstoff in Gegenwart eines Schwermetallhydrierungskatalysators, wie eines Nickel-, ferner Palladiumkatalysators, vorzugsweise auf einem geeigneten Trägermaterial, wie Calciumcarbonat oder Kohle, oder chemische Reduktionsmittel, wie reduzierende Schwermetalle, inkl. Schwermetallegierungen oder -amalgame, z.B. Zink, in Gegenwart eines Wasserstoffdonators, wie einer Säure, z.B. Essigsäure, oder eines Alkohols, z.B. Niederalkanols, reduzierende anorganische Salze, wie Alkalimetalljodide, z.B. Natriumjodid, oder Alkalimetallhydrogensulfite, z.B. Natriumhydrogensulfit, in Gegenwart eines Wasserstoffdonators, wie einer Säure, z.B. Essigsäure, oder Wasser, oder reduzierende organische Verbindungen, wie Ameisensäure, verwendet. Als Reduktionsmittel können auch Verbindungen zum Einsatz kommen, die leicht in entsprechende Epoxidverbindungen oder Oxide umgewandelt

werden können, wobei die Epoxidbildung aufgrund einer C,C-Doppelbindung und die Oxidbindung aufgrund eines vorhandenen Oxid-bildenden Hetero-, wie Schwefel-, Phosphor- oder Stickstoffatoms erfolgen kann. Solche Verbindungen sind z.B. geeignet substituierte Aethenverbindungen (die in der Reaktion in Aethylenoxidverbindungen umgewandelt werden), wie Tetracyanäthylen, oder insbesondere geeignete Sulfidverbindungen (die in der Reaktion in Sulfoxidverbindungen umgewandelt werden), wie Diniederalkylsulfide, in erster Linie Dimethylsulfid, geeignete organische Phosphorverbindungen, wie ein gegebenenfalls durch Phenyl und/oder Niederalkyl, z.B. Methyl, Aethyl, n-Propyl oder n-Butyl, substituiertes Phosphin (das in der Reaktion in ein Phosphinoxid umgewandelt wird), wie Triniederalkyl-phosphine, z.B. Tri-n-butylphosphin, oder Triphenylphosphin, ferner Triniederalkylphosphite (die in der Reaktion in Phosphorsäuretriniederalkylester übergeführt werden), üblicherweise in der Form von entsprechenden Alkoholadduktverbindungen, wie Trimethylphosphit, oder Phosphorigsäure-triamide, welche gegebenenfalls Niederalkyl als Substituenten enthalten, wie Hexaniederalkyl-phosphorigsäuretriamide, z.B. Hexamethylphosphorigsäuretriamid, letzteres vorzugsweise in der Form eines Methanoladdukts, ferner geeignete Stickstoffbasen (die in der Reaktion in die entsprechenden N-Oxide umgewandelt werden), wie heterocyclische Stickstoffbasen aromatischen Charakters, z.B. Basen vom Pyridintyp und insbesondere Pyridin selber. Die Spaltung des üblicherweise nicht isolierten Ozonids erfolgt normalerweise unter den Bedingungen, die man zu seiner Herstellung anwendet, d.h. in Gegenwart eines geeigneten Lösungsmittels oder Lösungsmittelgemisches, sowie unter Kühlen oder leichtem Erwärmen, wobei man vorzugsweise bei Temperaturen von etwa -10°C bis etwa +25°C arbeitet und die Reaktion üblicherweise bei Raumtemperatur abschliesst.

Stufe 2.4

Ein Azetidinon der Formel (VIa) kann hergestellt werden, indem man ein Oxalyl-azetidinon der Formel (XIV) solvolysiert.

Die Solvolyse kann als Hydrolyse, als Alkoholyse oder auch als Hydrazinolyse durchgeführt werden. Die Hydrolyse wird mit Wasser, gegebenenfalls in einem mit Wasser mischbaren Lösungsmittel, durchgeführt. Die Alkoholyse wird üblicherweise mit einem Niederalkanol, z.B. Methanol oder Aethanol, vorzugsweise in Gegenwart von Wasser und eines organischen Lösungsmittels, wie eines Niederalkancarbonsäure-niederalkylesters, z.B. Essigsäureäthylester, vorzugsweise bei Raumtemperatur, wenn notwendig unter Kühlen oder Erwärmen, z.B. bei einer Temperatur von etwa 0° bis etwa 80°C, durchgeführt. Die Hydrazinolyse wird auf konventionelle Weise mit einem substituierten Hydrazin, z.B. mit Phenyl- oder einem Nitrophenylhydrazin, wie 2-Nitrophenylhydrazin, 4-Nitrophenylhydrazin oder 2,4-Dinitrophenylhydrazin, das bevorzugt in etwa äquimolarer Menge eingesetzt wird, in einem organischen Lösungsmittel, wie einem Aether, z.B. Diäthyläther, einem aromatischen Kohlenwasserstoff, wie Benzol, einem halogenierten Kohlenwasserstoff, wie Methylenchlorid, einem Ester, wie Aethylacetat, und dergleichen, bei Temperaturen zwischen etwa Raumtemperatur und etwa 65°C durchgeführt.

In einer bevorzugten Ausführungsform des Verfahrens geht man von einer Verbindung der Formel (XIII) aus, die wie angegeben ozonisiert und dann reduktiv zum Oxalyl-azetidinon der Formel (XIV) gespalten wird, welches ohne Isolierung aus dem Reaktionsgemisch weiter zum Azetidinon der Formel (VIa) umgesetzt wird.

Bei der Ozonolyse entstehen gegebenenfalls geringe Mengen an Säure, welche die Abspaltung einer solvolytisch leicht abspaltbaren Hydroxyschutzgruppe R' im Rest $R_1$, z.B. eines trisubstituierten Silyl-Restes, bewirken können. Die dabei enstehende Verbindung der Formel

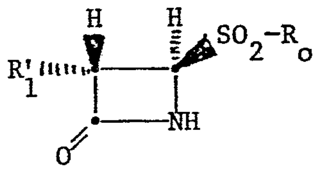

(VIb),

worin $R_1'$ durch Hydroxy substituiertes Niederalkyl ist, kann, beispielsweise chromatographisch, vom geschützten Azetidinon (VIa) abgetrennt und durch erneute Umsetzung mit dem die Hydroxyschutzgruppe R' einführenden Mittel der Formel $R'-X_3$ in das Azetidinon der Formel (VIa) übergeführt werden.

In den Verbindungen der Formeln (II), (II'), (III), (IV), (VII) bis (IX) und (XII) bis (XIV) kann eine geschützte Carboxylgruppe $R_2'$ nach an sich bekannten Methoden in eine andere geschützte Carboxylgruppe $R_2'$ übergeführt werden, wobei unter Berücksichtigung der gegebenenfalls in diesen Verbindungen enthaltenen weiteren funktionellen Gruppen die gleichen Methoden zur Anwendung gelangen können, wie zur Umwandlung dieses Substituenten in den Verbindungen der Formel (I) angegeben ist.

Die Erfindung betrifft ebenfalls neue Ausgangsprodukte sowie verfahrensgemäss erhältliche neue Zwischenprodukte, die diejenigen der Formeln (II) bis (IX) (incl. II', IIa und VIa) sowie die angegebenen Verfahren zur ihrer Herstellung.

Vorzugsweise werden solche Ausgangsstoffe verwendet und die Reaktionsbedingungen so gewählt, dass man zu den vorstehend als besonders bevorzugt aufgeführten Verbindungen gelangt.

Die Verbindungen der Formel I weisen wertvolle pharmakologischen Eigenschaften auf oder können als Zwischenprodukte zur Herstellung von solchen Verbindungen mit wertvollen pharmakologischen Eigenschaften verwendet werden. Verbindungen der Formel I, worin $R_1$ durch Hydroxy substituiertes Niederalkyl ist, $R_2$ Carboxyl oder eine unter physiologischen Bedingungen spaltbare veresterte Carboxylgruppe bedeutet, und $R_3$ und

A die unter Formel I angegebenen Bedeutungen haben und pharmakologisch annehmbaren Salze von solchen Verbindungen mit salzbildenden Gruppen haben antibakterielle Wirkungen. Beispielsweise sind sie in vitro gegen grampositive und gramnegative Kokken, z.B. Staphylococcus aureus, Streptococcus pyogenes, Streptococcus pneumoniae, Streptococcus faecalis, Neisseria meningitidis und Neisseria gonorrhoeae, gegen Enterobakterien, z.B. Escherichia coli und Proteus sp., gegen Haemophilus influenzae, Pseudomonas aeruginosa und Anaerobier, z.B. Bacteroides sp., in minimalen Konzentrationen von ca. 0,02 bis ca. 64 µg/ml wirksam. In vivo, bei der systemischen Infektion der Maus, z.B. durch Staphylococcus aureus, Escherichia coli oder Streptococcus pyogenes, ergeben sich bei subkutaner oder oraler Applikation $ED_{50}$-Werte von ca. 10 bis ca. 70 mg/kg.

Die neuen Verbindungen können als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, wobei eine geschützte Carboxylgruppe von einer physiologisch spaltbaren veresterten Carboxylgruppe verschieden ist, können als Zwischenprodukte zur Herstellung der oben genannten pharmakologisch wirksamen Verbindungen der Formel I verwendet werden.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Kalziumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B Mannit, enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 %, des Aktivstoffs.

Je nach Art der Infektion und Zustand des infizierten Organismus verwendet man tägliche Dosen von etwa 125 mg bis etwa 1,5 g zur oralen oder parenteralen Behandlung von Warmblütern (Menschen und Tiere) von etwa 70 kg Gewicht.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm,

IR: Infrarotspektrum,

UV: Ultraviolettspektrum,

NMR: Kernresonanzspektrum,

DBU: 1,5-Diazabicyclo[5.4.0]undec-5-en,

THF: Tetrahydrofuran,

DMF: Dimethylformamid.

## Experimenteller Teil

### Beispiel 1: (3R,S)-3-(Tetrazol-1-yl)-buttersäure

a) (3R,S)-(Tetrazol-1-yl)-buttersäure-methylester

10 g Tetrazol werden in 50 ml Crotonsäure-methylester gelöst und mit 10 Tropfen Pyridin versetzt. Das Reaktionsgemisch wird dann während 18 Stunden bei 90° gerührt. Nach Abkühlen wird 3 mal in Toluol aufgenommen und am Rotationsverdampfer eingeengt. Das Rohprodukt wird durch Säulenchromatographie gereinigt (Toluol/Aethylacetat 1:1), DC (Silicagel, Toluol/Aethylacetat 1:1): $R_f$ = 0,26.

b) (3R,S)-3-(Tetrazol-1-yl)-buttersäure

9,05 g (3R,S)-3-(Tetrazol-1-yl)-buttersäure-methylester werden mit 53,2 ml Essigsäure, 10,6 ml. konz. Salzsäure und 21,3 ml Wasser versetzt und 3 Stunden am Rückfluss erhitzt. Nach Abkühlen wird zur Trockene eingeengt. Der Rückstand wird dreimal mit Toluol am Wasserstrahlvakuum eingeengt und dann zweimal mit Aether digeriert. Nach Abfiltrieren wird der Feststoff am Hochvakuum getrocknet. NMR (DMSO $d_6$): $\delta$ = 9,55 (s, 1H), 7,9 (breit), 5,2 (m, 1H), 3,0 (d, 2H), 1,6 ppm (d, 3H).

### Beispiel 2: (3R,S)-3-(Tetrazol-1-yl)-buttersäurechlorid

2,34 g (3R,S)-3-(Tetrazol-1-yl)-buttersäure wird in 30 ml abs. Toluol mit 1,5 ml Thionylchlorid und 6 Tropfen abs. DMF versetzt und während 1 h bei 80° gerührt. Nach Abkühlen wird das Reaktionsgemisch am Rotationsverdampfer eingeengt, noch dreimal in Gegenwart von Methylenchlorid eingedampft und am Hochvakuum getrocknet. IR($CH_2Cl_2$): 1780 $cm^{-1}$.

### Beispiel 3: (3R,S)-3-(Pyrrol)-1-yl)-buttersäurechlorid

2,47 g (3R,S)-3-(Pyrrol-1-yl)-buttersäure [Chem. Pharm. Bull. 30, 2586 (1982)] wird in 30 ml abs. Methylenchlorid gelöst und mit

2, 64 ml 1-Dimethylamino-1-chlor-2-methylpropen versetzt. Das Reaktionsgemisch wird 2 Stunden 15 min. bei Raumtemperatur gerührt und ohne Isolierung weiter umgesetzt, wie in Beispiel 11 beschrieben.

Beispiel 4: 2-[(3S,4R)-3-(tert.Butyl-dimethylsilyloxymethyl)-4-triphenylmethylthio-2-oxo-azetidin-1-yl]-2-hydroxyessigsäure-allylester

8,4 g (3S,4R)-3-(tert.Butyldimethylsilyloxymethyl)-4-triphenylmethylthio-azetidin-2-on und 8,23 g Glyoxylsäureallylester-ethylhemiacetal in 170 ml abs. Toluol werden mit 27 g Molekularsieb (4 Å) versetzt und während 10 Stunden bei 55° gerührt. Nach Abfiltrieren und Einengen am Rotationsverdampfer unter vermindertem Druck wird das Rohprodukt durch Chromatographie an Silicagel gereinigt. (Laufmittel Toluol/Aethylacetat 95:5). DC (Silicagel, Toluol/Aethylacetat 10:1): $R_f$ = 0,37 und 0,27; IR ($CH_2Cl_2$): 3520, 1760, 1745 $cm^{-1}$.

Das Ausgangsmaterial (3S,4R)-3-(tert.Butyl-dimethylsilyloxymethyl)-4-triphenylmethylthio-azetidin-2-on kann wie folgt hergestellt werden:

a) (3S,5R,6R)-2,2-Dimethyl-6-(tert.-butyl-dimethylsilyloxymethyl)-penam-3-carbonsäuremethylester-1,1-dioxid

Eine Lösung von 23,6 g (85 mMol) (3S,5R,6R)-2,2-Dimethyl-6-hydroxymethyl-penam-3-carbonsäuremethylester-1,1-dioxid in 50 ml Dimethylformamid wird mit 25,5 g (170 mMol) tert.-Butyl-dimethylchlorsilan und 11,5 g (170 mMol) Imidazol bei Raumtemperatur während 45 min. gerührt. Dann wird das Lösungsmittel am Hochvakuum abdestilliert und der Rückstand in Aethylacetat aufgenommen. Die Lösung wird mit 1N-Schwefelsäure und dann mit Wasser gewaschen und die wässrigen Lösungen zweimal mit Aethylacetat extrahiert. Die organische Phase wird mit Natriumsulfat getrocknet und am Rotationsverdampfer eingeengt. Das Produkt fällt als kristalline Masse an.

DC Silicagel, Toluol/Aethylacetat (4:1): Rf = 0,56

IR ($CH_2Cl_2$) 3,4; 5,57; 5,65 µm.

b) 2-[(3S,4R)-3-(tert.-Butyl-dimethylsilyloxymethyl)-4-methylsulfonyl-2-oxo-azetidin-1-yl]-3-methyl-2-butensäuremethylester

Eine Lösung von 202 g (0,51 Mol) (3R,5R,6R)-2,2-Dimethyl-6-(tert.-butyl-dimethylsilyloxymethyl)-penam-3-carbonsäuremethylester-1,1-dioxid in 800 ml Tetrahydrofuran werden mit 9 ml DBU versetzt und während 5 Minuten bei Raumtemperatur gerührt. Dann werden weitere 95 ml DBU zugegeben und 30 Minuten bei Raumtemperatur gerührt. Anschliessend fügte man unter Kühlung 42,3 ml (0,68 Mol) Methyljodid zu. Nach 3 Stunden Reaktionsdauer wird vom auskristallisierten DBU-Hydrojodid abfiltriert und das Filtrat eingeengt. Der Rückstand wird in Aethylacetat aufgenommen und die Lösung in 1N-Schwefelsäure, Wasser und Natriumbicarbonatlösung gewaschen. Die wässerigen Phasen werden zweimal mit Aethylacetat extrahiert. Die vereinigten organischen Phasen werden über Natriumsulfat getrocknet und die Lösung zu einem dicken Oel eingeengt.

DC [Silicagel, Toluol/Aethylacetat (4:1)]; Rf = 0,42

IR ($CH_2Cl_2$) 5,63; 5,81; 6,17 µm.

c) (3S,4R)-3-Hydroxymethyl-4-methylsulfonyl-azetidin-2-on und (3S,4R)-3-(tert.Butyl-dimethylsilyloxymethyl)-4-methylsulfonyl-azetidin-2-on

Eine Lösung von 25 g (61,7 mMol) 2-[(3S,4R)-3-(tert.-Butyl-dimethylsilyloxymethyl)-4-methylsulfonyl-2-oxo-azetidin-1-yl]-3-methyl-2-butensäure-methylester in 400 ml Methylenchlorid wird bei -10° mit einem Ozon/Sauerstoffgemisch behandelt. Das Verschwinden des Ausgangsmaterials wird dünnschichtchromatographisch kontrolliert. Nach Beendigung der Reaktion werden 30 ml Dimethylsulfid zugegeben und für 3 Stunden bei Raumtemperatur weitergerührt. Die Lösung wird eingeengt und der Rückstand in einem Gemisch von 160 ml Methanol, 24 ml Aethylacetat und 3 ml Wasser aufgenommen und während 40 Minuten auf 70° erwärmt. Das Lösungsmittel wird anschliessend abgezogen und der Rückstand zweimal mit Toluol abgezogen. Das kristallisierende Oel wird in Methylenchlorid aufgenommen und die Kristalle, bestehend aus (3S,4R)-3-Hydroxymethyl-4-methylsulfonyl-azetidin-2-on, durch Filtration isoliert. Das Filtrat wird eingeengt und (3S,4R)-3-(tert.-Butyl-dimethylsilyloxymethyl)-4-methylsulfonyl-azetidin-2-on durch Chromatographie an Silicagel mit Toluol/Aethylacetat (3:1) in reiner Form erhalten:

(3S,4R)-3-Hydroxymethyl-4-methylsulfonyl-azetidin-2-on: DC, Silicagel, Toluol/Aethylacetat (1:1); Rf = 0,36, IR: ($CH_2Cl_2$) 2,96; 3,54; 5,61 µm.

(3S,4R)-3-(tert.Butyl-dimethylsilyloxymethyl)-4-methylsulfonyl-azetidin-2-on: DC; Silicagel, Toluol/Aethylacetat (1:1) Rf = 0,06.

Eine Lösung von 14,6 g (81,5 mMol) (3S,4R)-3-Hydroxymethyl-4-methylsulfonyl-azetidin-2-on in 40 ml Dimethylformamid wird mit 24 g (183 mMol) tert.-Butyldimethylchlorsilan und 11 g (163 mMol) Imidazol während 45 Minuten bei Raumtemperatur versetzt. Dann wird das Lösungsmittel am Hochvakuum abgezogen und der Rückstand in Aethylacetat aufgenommen. Die organische Phase wird nacheinander mit 1N-Schwefelsäure, Wasser und Natriumbicarbonatlösung gewaschen. Die wässerigen Phasen werden zweimal mit Aethylacetat extrahiert. Die vereinigten organischen Phasen werden über Natriumsulfat getrocknet und am Rotationsverdampfer eingeengt. Der kristalline Rückstand ist reines (3S,4R)-3-(tert.-Butyldimethylsilyloxymethyl)-4-methylsulfonyl-azetidin-2-on.

d) (3S,4R)-3-(tert.Butyldimethylsilyloxymethyl)-4-triphenylmethylthio-azetidin-2-on

12,5 g Triphenylmethylmercaptan werden in 70 ml Methanol bei 0° suspendiert, und über 10 Minuten portionsweise mit insgesamt 2,2 g einer 50 %igen Natriumhydrid-Suspension in Oel versetzt. Anschliessend wird eine Emulsion von 11,1 g 3-(tert.Butyl-dimethylsilyloxymethyl)-4-methylsulfonyl-azetidin-2-on in 70 ml Aceton und 70 ml Wasser über 30 min. zugetropft. Nach 30 min. Rühren bei 0° und 1 Stunde bei

Raumtemperatur wird das Reaktionsgemisch am Rotationsverdampfer eingeengt, mit Methylenchlorid versetzt, und die wässrige Phase wird abgetrennt. Die organische Lösung wird mit Sole gewaschen und über Natriumsulfat getrocknet. Nach Einengen wird die rohe Titelverbindung durch Chromathographie auf Silicagel (Laufmittel Toluol/Essigester 19:1) gereinigt. DC (Toluol-Essigester 19:1): $R_f$ = 0,64; IR (Methylenchlorid): 3390, 1760, 1117, 835 $cm^{-1}$.

Beispiel 5: 2-[(3S,4R)-3-(tert.Butyl-dimethylsilyloxymethyl)-4-triphenylmethylthio-2-oxo-azetidin-1-yl]-2-triphenylphosphoranylidenessigsäure-allylester

Zu einer Lösung von 604 mg 2-[(3S,4R)-3-(tert.Butyl-dimethylsilyloxymethyl)-4-triphenylmethylthio-2-oxo-azetidin-1-yl]-2-hydroxyessigsäure-allylester in 5 ml Tetrahydrofuran werden unter Rühren bei -15° nacheinander 80 µl Thionylchlorid und 88 µl Pyridin innert 5 min zugegeben. Die weisse Suspension wird 1 Stunde bei -10° nachgerührt und über Hyflo filtriert. Nach Waschen des Rückstands mit Toluol wird am Rotationsverdampfer eingeengt. Der Rückstand wird in 3 ml Dioxan gelöst, mit 293 mg Triphenylphosphin und 0,13 ml 2,6-Lutidin versetzt und während 2 Stunden bei 115° Badtemperatur gerührt. Das Gemisch wird über Hyflo filtriert und der Rückstand mit Toluol nachgewaschen. Die vereinten Filtrate werden eingedampft. Die Chromatographie des Rückstandes an Silicagel ergibt das reine Produkt (Laufmittel Toluol/Aethylacetat 95:5). DC (Silicagel, Toluol/Aethylacetat 1:1): $R_f$ = 0,18; IR ($CH_2Cl_2$): 1745, 1605 $cm^{-1}$.

Beispiel 6: Silbersalz des 2-[(3S,4R)-3-(tert.Butyl-dimethylsilyloxymethyl)-4-mercapto-2-oxo-azetidin-1-yl]-2-triphenylphosphoranylidenessigsäure-allylester

7,5 g 2-(3S,4R)-3-(tert.Butyl-dimethylsilyloxymethyl)-4-triphenylmethylthio-2-oxo-azetidin-1-yl]-2-triphenylphosphoranylidenessigsäure-allylester werden in 87 ml Aether vorgelegt und bei Raumtemperatur mit 70 ml einer 0,5 M wässrigen Silbernitrat-Lösung versetzt. Danach gibt

man tropfenweise ein Gemisch aus 3,6 ml Tributylamin, 0,18 ml Trifluoressigsäure und 25 ml Aether dazu und rührt das Reaktionsgemisch während 20 Minuten nach. Dann wird der Feststoff abgenutscht und mit Aether, Wasser und nochmals Aether gewaschen. Der Feststoff wird schliesslich zur Reinigung nochmals in 40 ml Aether und 40 ml Wasser aufgeschlemmt, abgenutscht und getrocknet. IR ($CH_2Cl_2$): 1760, 1620 $cm^{-1}$.

Beispiel 7: 2-[(3S,4R)-3-(tert.Butyl-dimethylsilyloxymethyl)-4-[(3R,S)-3-(tetrazol-1-yl)-butyroylthio]-2-oxo-azetidin-1-yl]-2-triphenylphosphoranylidenessigsäure-allylester

5 g Silbersalz des 2-[(3S,4R)-3-(tert.Butyl-dimethylsilyloxymethyl)-4-mercapto-2-oxo-azetidin-1-yl]-2-triphenylphosphoranylidenessigsäure-2-allylesters werden in 100 ml absolutem Methylenchlorid gelöst, auf 0° gekühlt und mit 85 mg 4-Dimethylaminopyridin und 1,23 ml Pyridin versetzt. Nach Zugabe von 1,9 g 3-(Tetrazol-1-yl)-buttersäurechlorid wird das Reaktionsgemisch 30 Minuten bei 0° gerührt. Der Niederschlag wird abfiltriert, das Filtrat mit Methylenchlorid verdünnt, mit wässriger $NaHCO_3$ Lösung und dann mit Sole gewaschen. Nach Trocknen über $Na_2SO_4$ wird die Lösung eingeengt und der Rückstand an Silicagel chromatographiert (Laufmittel Toluol-Aethylacetat). DC (Silicagel, Toluol-Aethylacetat 1:1): $R_f$ = 0,18; IR ($CH_2Cl_2$): 1750, 1680, 1610 $cm^{-1}$.

Beispiel 8: (5R,6S)-2-[(2R,S)-2-(Tetrazol-1-yl)-prop-1-yl]-6-(tert.butyl-dimethylsilyloxymethyl)-2-penem-3-carbonsäure-allylester

Eine Lösung von 3,4 g 2-[(3S,4R)-3-(tert.Butyl-dimethylsilyloxymethyl)-4-[(3R,S)-3-(tetrazol-1-yl)-butyroylthio]-2-oxo-azetidin-1-yl]-2-triphenylphosphoranylidenessigsäure-allylester in 500 ml absolutem Toluol wird unter Argonatmosphäre 2 Stunden bei Rückflusstemperatur gerührt. Dann wird das Lösungsmittel eingedampft und das Rohprodukt durch Chromatographie an Silicagel gereinigt (Laufmittel Toluol/Aethylacetat) 9:1). DC (Silicagel, Aethylacetat); $R_f$ = 0,55; IR ($CH_2Cl_2$): 1780, 1695, 1575 $cm^{-1}$.

Beispiel 9: (5R,6S)-2-[(2R,S)-2-(Tetrazol-1-yl)-prop-1-yl]-6-hydroxymethyl-2-penem-3-carbonsäure-allylester

Eine Lösung von 1,5 g (5R,6S)-2-[(2R,S)-2-(Tetrazol-1-yl)-prop-1-yl]-6-(tert.butyl-dimethylsilyloxymethyl)-2-penem-3-carbonsäure-allylester in 32 ml abs. THF wird auf -70° abgekühlt und nacheinander mit 1,31 ml Essigsäure und tropfenweise über 15 Minuten mit 70 ml einer 0,1 M Tetrabutylammoniumfluorid-Lösung in THF versetzt. Das Kühlbad wird dann entfernt und das Reaktionsgemisch langsam auf Raumtemperatur gebracht. Nach 5 stündigem Rühren bei Raumtemperatur wird das Reaktionsgemisch am Rotationsverdampfer konzentriert und in Aethylacetat und wässriger $NaHCO_3$ aufgenommen. Die organische Phase wird abgetrennt, mit Sole gewaschen über $Na_2SO_4$ getrocknet und eingeengt. Das Rohprodukt wird durch Chromatographie auf Silicagel gereinigt (Laufmittel Toluol-Aethylacetat 4:1 bis 1:1). DC (Silicagel, Aethylacetat): $R_f$ = 0,22; IR ($CH_2Cl_2$): 3600, 1780, 1695, 1575 $cm^{-1}$.

Beispiel 10: (5R,6S)-2-[(2R,S)-2-(Tetrazol)-1-yl)-prop-1-yl]-6-hydroxymethyl-2-penem-3-carbonsäure, Natriumsalz

0,85 g (5R,6S)-2-[(2R,S)-(Tetrazol-1-yl)-prop-1-yl]-6-hydroxymethyl-2-penem-3-carbonsäure-allylester werden in 32 ml abs. THF gelöst, auf -10° abgekühlt und mit 53 mg Tetrakis-triphenylphosphin-palladium und 0,75 ml Tributylzinnhydrid versetzt. Nach 35 Minuten Rühren bei -10° werden 0,16 ml Essigsäure zugegeben und das Reaktionsgemisch bei -5° 10 Minuten weitergerührt. Nach Konzentrieren am Rotationsverdampfer wird der Rückstand in Wasser/Aethylacetat aufgenommen, gekühlt und mit $NaHCO_3$ auf pH 7,5 gestellt. Die wässrige Phase wird abgetrennt, 2 mal mit Aethylacetat gewaschen, am Rotationsverdampfer konzentriert und auf einer XAD-2 Säule gereinigt (Laufmittel: Wasser). Die vereinigten Fraktionen werden am Hochvakuum lyophilisiert.
DC (Reversed-Phase Opti UPC 12, Wasser): $R_f$ = 0,3; UV (Phosphatpuffer pH 7,4): $\lambda$max. = 304 nm.

Beispiel 11: 2-[(3S,4R)-3-(tert.Butyldimethylsilyloxymethyl)-4-[(3R,S)-3-(pyrrol-1-yl)]-butyroylthio]-2-oxo-azetidin-1-yl]-2-triphenylphosphoranylidenessigsäure-allylester

Analog Beispiel 7 werden 5 g Silbersalz des 2-[(3S,4R)-3-(tert.Butyldimethylsilyloxymethyl)-4-mercapto-2-oxo-azetidin-1-yl]-2-triphenylphosphoranyliden-essigsäure-2-allylesters und 1,5 Moläquivalent 3-(Pyrrol-1-yl)-buttersäurechlorid zur Titelverbindung umgesetzt. DC (Silicagel, Aethylacetat): $R_f$ = 0,62; IR ($CH_2Cl_2$): 1750, 1680, 1625 $cm^{-1}$.

Beispiel 12: (5R,6S)-2-[(2R,S)-2-(Pyrrol-1-yl)-prop-1-yl]-6-(tert.-butyl-dimethylsilyloxymethyl)-2-penem-3-carbonsäure-allylester

Analog Beispiel 8 werden 3,86 g 2-[(3S,4R)-3-(tert.Butyl-dimethylsilyloxymethyl)-4-[(3R,S)-3-(pyrrol-1-yl)-butyroylthio]-2-oxo-azetidin-1-yl]-2-triphenylphosphoranylidenessigsäure-allylester in die Titelverbindung überführt. DC (Silicagel, Toluol/Aethylacetat 1:1): $R_f$ = 0,7; IR ($CH_2Cl_2$): 1780, 1700, 1575 $cm^{-1}$.

Beispiel 13: (5R,6S)-2-[(2R,S)-2-(Pyrrol-1-yl)-prop-1-yl]-6-hydroxymethyl-2-penem-3-carbonsäure-allylester

Analog Beispiel 9 werden 1,5 g (5R,6S)-2-[(2R,S)-2-(Pyrrol-1-yl)-prop-1-yl]-6-(tert.butyl-dimethylsilyloxymethyl)-2-penem-3-carbonsäure-allylester in die Titelverbindung überführt. DC (Silicagel, Toluol/Aethylacetat 1:1): $R_f$ = 0,35; IR ($CH_2Cl_2$): 3600, 1780, 1695, 1575 $cm^{-1}$.

Beispiel 14: (5R,6S)-2-[(2R,S)-2-(Pyrrol-1-yl)-prop-1-yl]-6-hydroxymethyl-2-penem-3-carbonsäure, Natriumsalz

Analog Beispiel 10 werden 1 g (5R,6S)-2-[(2R,S)-2-Pyrrol-1-yl)-prop-1-yl]-6-hydroxymethyl-2-penem-3-carbonsäure-allyester in die Titelverbindung überführt. DC (Reversed Phase; Opti UPC 12, Wasser-Acetonitril 4:1): $R_f$ = 0,38; UV (Wasser): $\lambda$max. = 308 nm.

Beispiel 15: (3R,S)-3-(Tetrazol-1-yl)-thiobuttersäure

2 g (3R,S)-3-(Tetrazol-1-yl)-buttersäurechlorid in 4 ml abs. Methylenchlorid wird bei 0° zu einem Ueberschuss einer Pyridin/Schwefelwasserstoff-Lösung in Methylenchlorid gegeben. Das Reaktionsgemisch wird eine Stunde gerührt bei 0°, mit Chloroform verdünnt und mit 2N Schwefelsäure sauer gestellt. Die wässrige Lösung wird noch zweimal mit Chloroform extrahiert. Die vereinten organischen Phasen werden mit wässriger $NaHCO_3$-Lösung extrahiert. Nach Ansäuern der Extrakte mit $H_2SO_4$ (2N) und mehrmaligem Waschen mit Chloroform wird die organische Lösung über $Na_2SO_4$ getrocknet und zur Trockene eingedampft. Man erhält die Titelverbindung. IR ($CH_2Cl_2$): 2560, 1695 $cm^{-1}$.

Beispiel 16: (3S,4R)-3-(tert.Butyldimethylsilyloxymethyl)-4-[(3R,S)-3-tetrazol-1-yl)-butyroylthio]-azetidin-2-on

8,2 g (3S,4R)-3-(tert.Butyldimethylsilyloxymethyl)-4-methylsulfonyl-azetidin-2-on und 6,7 g (3R,S)-3-(Tetrazol-1-yl)-thiobuttersäure werden in 180 ml Methylenchlorid gelöst und mit 180 ml Wasser und 42 ml 1N NaOH versetzt. Die Emulsion wird während 1,5 Stunden bei Raumtemperatur kräftig gerührt. Die organische Phase wird abgetrennt und die wässrige Phase noch zweimal mit $CH_2Cl_2$ extrahiert. Die vereinigten organischen Extrakte werden mit einer gesättigten wässrigen $NaHCO_3$-Lösung, dann mit Sole gewaschen, über $Na_2SO_4$ getrocknet und eingedampft. Das erhaltene Rohprodukt wird durch Chromatographie an Silicagel gereinigt. IR ($CH_2Cl_2$): 3425, 1765, 1685 $cm^{-1}$.

Die Herstellung des Ausgangsmaterials (3S,4R)-3-(tert.-Butyldimethylsilyloxymethyl)-4-methylsulfonyl-azetidin-2-on ist im Beispiel 4 beschrieben.

Beispiel 17: 2-[(3S,4R)-3-(tert.Butyl-dimethylsilyloxymethyl)-4-[(3R,S)-3-(tetrazol-1-yl)-butyroylthio]-2-oxo-azetidin-1-yl]-2-hydroxyessigsäure-allylester

Analog Beispiel 4 werden 8,1 g (3S,4R)-3-(tert.Butyl-dimethylsilyloxymethyl)-4-[(3R,S)-3-(tetrazol-1-yl)-butyroylthio]-azetidin-2-on zur Titelverbindung umgesetzt. IR ($CH_2Cl_2$): 3515, 1760, 1745, 1685 $cm^{-1}$.

Beispiel 18: 2-[(3S,4R)-3-(tert.Butyl-dimethylsilyloxymethyl)-4-[(3R,S)-3-(tetrazol-1-yl)-butyroylthio]-2-oxo-azetidin-1-yl]-2-triphenylphosphoranylidenessigsäure-allylester

Analog Beispiel 5 werden 7,43 g 2-[(3S,4R)-3-(tert.Butyl-dimethylsilyloxymethyl)-4-[(3R,S)-3-(tetrazol-1-yl)-butyroylthio]-2-oxo-azetidin-1-yl]-2-hydroxyessigsäure-allylester zur Titelverbindung umgesetzt. DC (Silicagel, Toluol/Aethylacetat 1:1): $R_f$ = 0,18; IR ($CH_2Cl_2$): 1750, 1680, 1610 $cm^{-1}$. Das Produkt ist mit demjenigen von Beispiel 7 identisch.

Beispiel 19: In analoger Weise, wie in den vorhergehenden Beispielen beschrieben, erhält man die folgenden Verbindungen:

(5R,6S)-2-[(2R,S)-2-(Tetrazol-1-yl)-but-1-yl]-6-hydroxymethyl-2-penem-3-carbonsäure, Natriumsalz, UV (Wasser): $\lambda$ max. = 306 nm;

(5R,6S)-2-[(2R,S)-2-(1,2,4-Triazol-1-yl)-prop-1-yl]-6-hydroxymethyl-2-penem-3-carbonsäure, Natriumsalz, UV (Phosphatpuffer pH 7.4) $\lambda$ max = 303 nm;

(5R,6S)-2-[(2R,S)-2-(5-Amino-tetrazol-1-yl)-prop-1-yl]-6-hydroxymethyl-2-penem-3-carbonsäure, UV (Wasser): $\lambda$ max = 305 nm;

(5R,6S)-2-[(2R,S)-2-(Imidazol-1-yl)-prop-1-yl]-6-hydroxymethyl-2-penem-3-carbonsäure, Natriumsalz, UV (Wasser): $\lambda$ max = 308 nm;

(5R,6S)-2-[(2R,S)-2-(Pyrazol-1-yl)-prop-1-yl]-6-hydroxymethyl-2-penem-3-carbonsäure, Natriumsalz, UV (Wasser): $\lambda$ max = 309 nm;

(5R,6S)-2-[(1R,S)-1-(Tetrazol-1-yl)-ethyl]-6-hydroxymethyl-2-penem-3-carbonsäure, Natriumsalz, UV (Phosphatpuffer pH 7.4): λ max. = 305 nm;

(5R,6S)-2-[(2R,S)-2-(Tetrazol-1-yl)-prop-1-yl]-6-[(1R)-hydroxyäthyl]-2-penem-3-carbonsäure, Natriumsalz, UV (Wasser): λ max. = 306 nm, und

(5R,6S)-2-[(2R,S)-2-(Tetrazol-1-yl)-prop-1-yl]-6-(2-hydroxy-prop-2-yl)-2-penem-3-carbonsäure, Natriumsalz, UV Wasser): λ max. = 307 nm.

Beispiel 20: (5R,6S)-2-[(2R,S)-2-(Tetrazol-1-yl)-prop-1-yl]-6-hydroxymethyl-2-penem-3-carbonsäure-1-äthoxycarbonyloxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 15,0 ml Methylenchlorid getropft und von den ausgefallenen anorganischen Salzen abfiltriert. Die Methylenchloridlösung wird bis auf 2 ml eingeengt und bei 0° zu einer Lösung von 0,332 g (1 mMol) (5R,6S)-2-[(2R,S)-2-(Tetrazol-1-yl)-prop-1-yl]-6-hydroxymethyl-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): Absorptionsbanden bei 1788 und 1740 $cm^{-1}$.

Beispiel 21: In analoger Weise, wie in Beispiel 20 beschrieben, erhält man ausgehend von (5R,6S)-2-[(2R,S)-2-(Pyrrol-1-yl)-prop-1-yl]-6-hydroxymethyl-2-penem-3-carbonsäure den (5R,6S)-2-[(2R,6S)-2-(Pyr-

rol-1-yl)-prop-1-yl]-6-hydroxymethyl-2-penem-3-carbonsäure-1-äthoxycarbonyloxyäthylester. IR Spektrum (Methylenchlorid): 1786 und 1743 $cm^{-1}$.

Beispiel 22: (5R,6S)-2-[(2R,S)-2-(Tetrazol-1-yl)-prop-1-yl]-6-hydroxymethyl-2-penem-3-carbonsäure-pivaloyloxymethylester

0,6 g Natriumjodid werden in 2 ml Aceton gelöst und mit 0,15 ml Pivalinsäurechlormethylester versetzt. Das Gemisch wird bei Raumtemperatur während 3 Stunden gerührt und anschliessend auf 7,5 ml Methylenchlorid getropft. Die ausgefallenen anorganischen Salze werden abfiltriert. Die Methylenchloridlösung wird bis auf 1 ml eingeengt und zu einer Lösung von 0,133 g (0,4 mMol) (5R,6S)-2-[(2R,S)-2-(Tetrazol-1-yl)-prop-1-yl]-6-hydroxymethyl-2-penem-3-carbonsäure und 0,07 ml Diisopropyläthylamin in 4 ml N,N-Dimethylacetamid bei 0° gegeben. Dann wird während 3 Stunden bei 0° gerührt, anschliessend mit Aethylacetat verdünnt und dreimal mit Wasser gewaschen. Die organische Phase wird über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): Absorptionsbanden bei 1790 und 1730.

Beispiel 23: In analoger Weise, wie in Beispiel 22 beschrieben, erhält man ausgehend von (5R,6S)-2-[(2R,S)-2-(Pyrrol-1-yl)-prop-1-yl]-6-hydroxymethyl-2-penem-3-carbonsäure den (5R,6S)-2-[(2R,S)-2-(Pyrrol-1-yl)-prop-1-yl]-6-hydroxymethyl-2-penem-3-carbonsäure-pivaloyloxymethylester. IR-Spektrum (Methylenchlorid): 1789 und 1726 $cm^{-1}$.

Beispiel 24: Trockenampullen oder Vials, enthaltend 0,5 g Natriumsalz der (5R,6S)-2-[(2R,S)-2-(Tetrazol-1-yl)-prop-1-yl]-6-hydroxymethyl-2-penem-3-carbonsäure als Wirkstoff, werden wie folgt hergestellt:

Zusammensetzung (für 1 Ampulle oder Vial):

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des oben genannten Wirkstoffs kann auch dieselbe Menge eines anderen Wirkstoffs der vorangehenden Beispiele, wie z.B. das Natriumsalz der (5R,6S)-2-[(2R,S)-2-(Pyrrol-1-yl)-prop-1-yl]-6-hydroxymethyl-2-penem-3-carbonsäure, verwendet werden.

Patentansprüche (für alle benannten Vertragsstaaten ausser Oesterreich)

1. 2-Heterocyclylniederalkyl-2-penem-Verbindungen der Formel

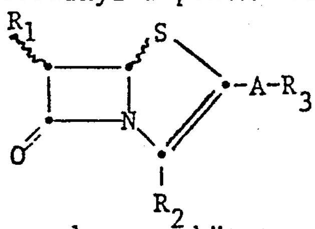

(I),

worin $R_1$ durch Hydroxy oder geschütztes Hydroxy substituiertes Niederalkyl ist, $R_2$ Carboxyl oder funktionell abgewandeltes Carboxyl bedeutet, $R_3$ einen über ein tertiäres Ringstickstoffatom an den Rest -A- gebundenen, ungesättigten monocyclischen Azaheterocyclyl-Rest darstellt und A durch Niederalkyl substituiertes geradkettiges Niederalkylen bedeutet, optische Isomere von Verbindungen der Formel (I), Mischungen dieser optischen Isomere und Salze von solchen Verbindungen der Formel (I), die eine salzbildende Gruppe aufweisen.

2. Verbindungen der Formel I gemäss Patentanspruch 1, worin $R_1$ durch Hydroxy oder geschütztes Hydroxy substituiertes Niederalkyl ist, $R_2$ Carboxyl, unter physiologischen Bedingungen spaltbares verestertes Carboxyl oder geschütztes Carboxyl $R_2'$ bedeutet, $R_3$ einen über ein tertiäres Ringstickstoffatom an den Rest -A- gebundenen monocyclischen, gegebenenfalls partiell gesättigten 5-gliedrigen Heteroaryl-Rest mit 1-4 Ringstickstoffatomen, wie einen entsprechenden aza-, diaza-, triaza- oder tetraza-cyclischen Rest aromatischen Charakters oder einen entsprechenden Dihydro-Rest, oder einen entsprechenden partiell gesättigten 6-gliedrigen Heteroaryl-Rest mit 1-3 Ringstickstoffatomen, wie einen entsprechenden aza-, diaza- oder triaza-cyclischen Rest, z.B. einen entsprechenden Dihydro- oder Tetrahydro-Rest, darstellt, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, Aminoniederalkyl, Diniederalkylaminoniederalkyl, Sulfoniederalkyl, Amino, Niederalkylamino, Diniederalkylamino, Niederalkylenamino, Niederalkanoylamino, Carboxy, Niederalkoxycarbonyl, gegebenenfalls N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Oxo und/oder Oxido substituiert sind, und A durch Niederalkyl substituiertes geradkettiges Niederalkylen bedeutet, optische Isomere von Verbindungen der Formel (I), Mischungen dieser Isomere und Salze von Verbindungen der Formel (I), die eine salzbildende Gruppe aufweisen.

3. Verbindungen der Formel I gemäss Patentanspruch 1, worin $R_1$ durch Hydroxy oder Triniederalkylsiloxy substituiertes Niederalkyl ist, $R_2$ Carboxyl, Niederalkenyloxycarbonyl, gegebenenfalls durch Nitro substituiertes Benzyloxycarbonyl, 2-Triniederalkylsilyläthoxycarbonyl oder eine unter physiologischen Bedingungen spaltbare veresterte Carboxylgruppe, z.B. 1-Niederalkoxycarbonyloxyniederalkoxycarbonyl, Niederalkanoyloxymethoxycarbonyl, α-Aminoniederalkanoyloxymethoxycarbonyl oder Phthalidyloxycarbonyl, bedeutet, $R_3$ über ein tertiäres Ringstickstoffatom an den Rest -A- gebundenes, unsubstituiertes oder durch Niederalkyl oder Halogen substituiertes Pyrrolyl, z.B. 1-Pyrrolyl, unsubstituiertes oder durch Niederalkyl substituiertes Imidazolyl oder Pyrazolyl, z.B. 1-Imidazolyl oder 1-Pyrazolyl, unsubstituiertes oder durch Niederalkyl, Carboxyniederalkyl oder Phenyl substituiertes Triazolyl, z.B. 1H-1,2,3-Triazol-1-yl, 2H-1,2,3-Triazol-2-yl, 1H-1,2,4-Triazol-1-yl oder 1H-1,3,4-Triazol-1-yl, unsubstituiertes oder durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolyl, wie 1H-Tetrazol-1-yl oder 2H-Tetrazol-2-yl, unsubstituiertes oder durch Oxo und gegebenenfalls zusätzlich durch Halogen substituiertes Dihydro-1-pyridyl, z.B. 2H-1,2-Dihydro-1-pyridyl oder 4H-1,4-Dihydro-1-pyridyl, unsubstituiertes oder durch Oxo und gegebenenfalls zusätzlich durch Niederalkyl, Amino, Diniederalkylamino oder Carboxy substituiertes Dihydro-1-pyrimidyl, z.B. 2H-1,2-Dihydro-1-pyrimidyl

oder 4H-1,4-Dihydro-1-pyrimidyl, oder unsubstituiertes oder durch Niederalkyl und/oder bis zu zwei Oxo substituiertes Dihydro- oder Tetrahydrotriazinyl, z.B. 2H-1,2-Dihydro-1,3,5-triazin-1-yl, 2H-1,2-Dihydro-1,2,4-triazin-1-yl, 2H-1,2,5,6-Tetrahydro-1,2,4-triazin-1-yl oder 4H-1,4,5,6-Tetrahydro-1,2,4-triazin-1-yl, bedeutet, und A durch Niederalkyl substituiertes geradkettiges Niederalkylen darstellt, optische Isomere von Verbindungen der Formel (I), Mischungen dieser optischen Isomere und Salze, insbesondere phamazeutisch annehmbare Salze von solchen Verbindungen der Formel (I), die eine salzbildende Gruppe enthalten.

4. Verbindungen der Formel I gemäss Patentanspruch 1, worin $R_1$ durch Hydroxy substituiertes Niederalkyl ist, $R_2$ Carboxyl, 1-Niederalkoxycarbonyloxyniederalkoxycarbonyl oder Niederalkanoyloxymethoxycarbonyl bedeutet, $R_3$ über ein tertiäres Stickstoffatom an den Rest -A- gebundenes Pyrrolyl, Imidazolyl, z.B. 1-Imidazolyl, 1H-Triazolyl, z.B. 1H-1,2,4-Triazol-1-yl, oder unsubstituiertes oder durch Amino, Carboxyniederalkyl, Sulfoniederalkyl oder Diniederalkylaminoniederalkyl substituiertes 1H-Tetrazolyl, z.B. 1H-Tetrazol-1-yl oder 2H-Tetrazol-2-yl, darstellt, und A durch Niederalkyl mit 1-2 Kohlenstoffatomen mono- oder di-substituiertes Niederalkylen mit 1-4 Kohlenstoffatomen bedeutet, optische Isomere von Verbindungen der Formel (I), z.B. das (5R,6S)-Isomere, Mischungen dieser optischen Isomere und pharmazeutisch annehmbare Salze von solchen Verbindungen der Formel (I), die eine salzbildende Gruppe aufweisen.

5. (5R,6S)-Konfigurierte Verbindungen der Formel I gemäss Patentanspruch 1, worin $R_1$ Hydroxymethyl oder 1-Hydroxyäthyl bedeutet, $R_2$ Carboxyl ist, $R_3$ Pyrrol-1-yl oder 1H-Tetrazol-1-yl darstellt und A 1,2-Propylen ist, und pharmazeutisch annehmbare Salze davon.

6. (5R,6S)-2-[(2R,S)-2-(Pyrrol-1-yl)-prop-1-yl]-6-hydroxymethyl-2-penem-3-carbonsäure und pharmazeutisch annehmbare Salze davon gemäss Patentanspruch 1.

7. (5R,6S)-2-[(2R,S)-2-(Tetrazol)-1-yl)-prop-1-yl]-6-hydroxymethyl-2-penem-3-carbonsäure und pharmazeutisch annehmbare Salze davon gemäss Patentanspruch 1.

8. Pharmazeutische Präparate enthaltend Verbindungen der Formel I oder pharmazeutisch annehmbare Salze von solchen Verbindungen gemäss Patentanspruch 1.

9. Verwendung von Verbindungen der Formel I oder von pharmazeutisch annehmbaren Salzen von solchen Verbindungen gemäss Patentanspruch 1 zur Herstellung von pharmazeutischen Präparaten.

10. Verbindungen der Formel I und pharmazeutisch annehmbare Salze von solchen Verbindungen gemäss Patentanspruch 1 zur Anwendung in einem Verfahren zur therapeutischen Behandlung des menschlichen oder tierischen Körpers.

11. Verbindungen der Formel I und pharmazeutisch annehmbare Salze von solchen Verbindungen gemäss Patentanspruch 1 als antibiotische Mittel.

12. Verfahren zur Herstellung von Verbindungen der Formel I gemäss Patentanspruch 1, ihren optischen Isomeren, Mischungen ihrer optischen Isomere und ihren Salzen, dadurch gekennzeichnet, dass man eine Ylid-Verbindung der Formel

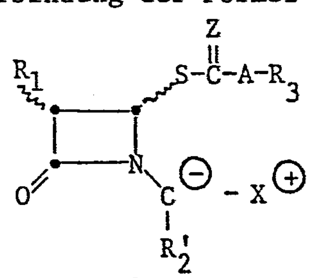

(II),

worin $R_1$, $R_3$ und A die unter Formel (I) angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe

zusammen mit einem Kation darstellt, ringschliesst, oder eine Verbindung der Formel

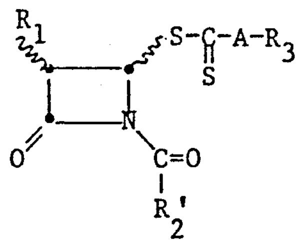

(III),

worin $R_1$, $R_3$ und A die unter Formel (I) angegebenen Bedeutungen haben, und $R_2'$ eine geschützte Carboxylgruppe darstellt, mit einer organischen Verbindung des dreiwertigen Phosphors behandelt, oder eine Verbindung der Formel

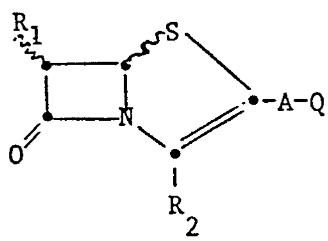

(IV),

worin $R_1$, $R_2$ und A die unter Formel (I) angegebenen Bedeutungen haben und Q eine reaktionsfähige veresterte Hydroxygruppe bedeutet, mit einem den Azaheterocyclyl-Rest $R_3$ einführenden Mittel umsetzt,

und, wenn erwünscht oder notwendig in einer erhältlichen Verbindung der Formel (I) eine geschützte Hydroxygruppe im Rest $R_1$ in die freie Hydroxygruppe überführt und/oder, wenn erwünscht, in einer erhältlichen Verbindung der Formel (I) eine geschützte Carboxylgruppe $R_2'$ in die freie, in eine unter physiologischen Bedingungen spaltbare veresterte Carboxylgruppe oder in eine andere geschütze Carboxylgruppe $R_2'$ überführt, und/oder, wenn erwünscht, eine freie Carboxylgruppe $R_2$ in eine unter physiologischen Bedingungen spaltbare veresterte Carboxylgruppe überführt, und/oder, wenn erwünscht weitere im Rest $R_3$ enthaltene geschützte funktionelle Gruppen in die freien funktionellen Gruppen überführt, und/oder, wenn erwünscht, in einer erhältlichen Verbindung der Formel (I) einen Rest $R_3$ in einen

anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

13. Die nach dem Verfahren gemäss Patentanspruch 12 erhältliche Verbindungen.

Patentansprüche (für Oesterreich)

1. Verfahren zur Herstellung von 2-Heterocyclylniederalkyl-2-penem-Verbindungen der Formel

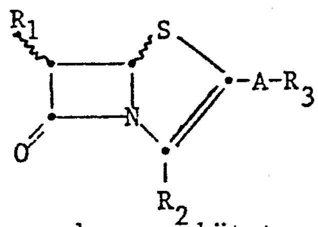

(I),

worin $R_1$ durch Hydroxy oder geschütztes Hydroxy substituiertes Niederalkyl ist, $R_2$ Carboxyl oder funktionell abgewandeltes Carboxyl bedeutet, $R_3$ einen über ein tertiäres Ringstickstoffatom an den Rest -A- gebundenen, ungesättigten monocyclischen Azaheterocyclyl-Rest darstellt und A durch Niederalkyl substituiertes geradkettiges Niederalkylen bedeutet, optische Isomere von Verbindungen der Formel (I), Mischungen dieser optischen Isomere und Salze von solchen Verbindungen der Formel (I), die eine salzbildende Gruppe aufweisen, dadurch gekennzeichnet, dass man eine Ylid-Verbindung der Formel

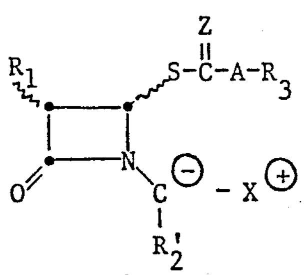

(II),

worin $R_1$, $R_3$ und A die unter Formel (I) angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

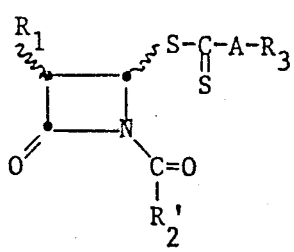

(III),

worin $R_1$, $R_3$ und A die unter Formel (I) angegebenen Bedeutungen haben, und $R_2'$ eine geschützte Carboxylgruppe darstellt, mit einer organischen Verbindung des dreiwertigen Phosphors behandelt, oder eine Verbindung der Formel

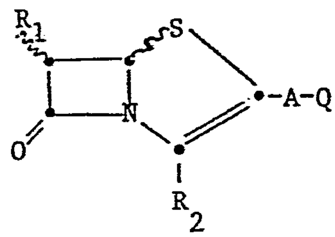

(IV),

worin $R_1$, $R_2$ und A die unter Formel (I) angegebenen Bedeutungen haben und Q eine reaktionsfähige veresterte Hydroxygruppe bedeutet, mit einem den Azaheterocyclyl-Rest $R_3$ einführenden Mittel umsetzt,

und, wenn erwünscht oder notwendig in einer erhältlichen Verbindung der Formel (I) eine geschützte Hydroxygruppe im Rest $R_1$ in die freie Hydroxygruppe überführt und/oder, wenn erwünscht, in einer erhältlichen Verbindung der Formel (I) eine geschützte Carboxylgruppe $R_2'$ in die freie, in eine unter physiologischen Bedingungen spaltbare veresterte Carboxylgruppe oder in eine andere geschütze Carboxylgruppe $R_2'$ überführt, und/oder, wenn erwünscht, eine freie Carboxylgruppe $R_2$ in eine unter physiologischen Bedingungen spaltbare veresterte Carboxylgruppe überführt, und/oder, wenn erwünscht weitere im Rest $R_3$ enthaltene geschützte funktionelle Gruppen in die freien funktionellen Gruppen überführt, und/oder, wenn erwünscht, in einer erhältlichen Verbindung der Formel (I) einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

2. Verfahren zur Herstellung von Verbindungen der Formel I gemäss Patentanspruch 1, worin $R_1$ durch Hydroxy oder geschütztes Hydroxy substituiertes Niederalkyl ist, $R_2$ Carboxyl, unter physiologischen Bedingungen spaltbares verestertes Carboxyl oder geschütztes Carboxyl $R_2'$ bedeutet, $R_3$ einen über ein tertiäres Ringstickstoffatom an den Rest -A- gebundenen monocyclischen, gegebenenfalls partiell gesättigten 5-gliedrigen Heteroaryl-Rest mit 1-4 Ringstickstoffatomen, wie einen entsprechenden aza-, diaza-, triaza- oder tetraza-cyclischen Rest aromatischen Charakters oder einen entsprechenden Dihydro-Rest, oder einen entsprechenden partiell gesättigten 6-gliedrigen Heteroaryl-Rest mit 1-3 Ringstickstoffatomen, wie einen entsprechenden aza-, diaza- oder triaza-cyclischen Rest, z.B. einen entsprechenden Dihydro- oder Tetrahydro-Rest, darstellt, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, Aminoniederalkyl, Diniederalkylaminoniederalkyl, Sulfoniederalkyl, Amino, Niederalkylamino, Diniederalkylamino, Niederalkylenamino, Niederalkanoylamino, Carboxy, Niederalkoxycarbonyl, gegebenenfalls N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Oxo und/oder Oxido substituiert sind, und A durch Niederalkyl substituiertes geradkettiges Niederalkylen bedeutet, von optischen Isomeren von Verbindungen der Formel I, von Mischungen dieser optischen Isomere, und von Salzen von Verbindungen der Formel I, die eine salzbildende Gruppe aufweisen.

3. Verfahren zur Herstellung von Verbindungen der Formel I, gemäss Patentanspruch 1, worin $R_1$ durch Hydroxy oder Triniederalkylsiloxy substituiertes Niederalkyl ist, $R_2$ Carboxyl, Niederalkenyloxycarbonyl, gegebenenfalls durch Nitro

substituiertes Benzyloxycarbonyl, 2-Triniederalkylsilyläthoxycarbonyl oder eine unter physiologischen Bedingungen spaltbare veresterte Carboxylgruppe, z.B. 1-Niederalkoxycarbonyloxyniederalkoxycarbonyl, Niederalkanoyloxymethoxycarbonyl, α-Aminoniederalkanoyloxymethoxycarbonyl oder Phthalidyloxycarbonyl, bedeutet, $R_3$ über ein tertiäres Ringstickstoffatom an den Rest -A- gebundenes, unsubstituiertes oder durch Niederalkyl oder Halogen substituiertes Pyrrolyl, z.B. 1-Pyrrolyl, unsubstituiertes oder durch Niederalkyl substituiertes Imidazolyl oder Pyrazolyl, z.B. 1-Imidazolyl oder 1-Pyrazolyl, unsubstituiertes oder durch Niederalkyl, Carboxyniederalkyl oder Phenyl substituiertes Triazolyl, z.B. 1H-1,2,3-Triazol-1-yl, 2H-1,2,3-Triazol-2-yl, 1H-1,2,4-Triazol-1-yl oder 1H-1,3,4-Triazol-1-yl, unsubstituiertes oder durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolyl, wie 1H-Tetrazol-1-yl oder 2H-Tetrazol-2-yl, unsubstituiertes oder durch Oxo und gegebenenfalls zusätzlich durch Halogen substituiertes Dihydro-1-pyridyl, z.B. 2H-1,2-Dihydro-1-pyridyl oder 4H-1,4-Dihydro-1-pyridyl, unsubstituiertes oder durch Oxo und gegebenenfalls zusätzlich durch Niederalkyl, Amino, Diniederalkylamino oder Carboxy substituiertes Dihydro-1-pyrimidyl, z.B. 2H-1,2-Dihydro-1-pyrimidyl oder 4H-1,4-Dihydro-1-pyrimidyl, oder unsubstituiertes oder durch Niederalkyl und/oder bis zu zwei Oxo substituiertes Dihydro- oder Tetrahydrotriazinyl, z.B. 2H-1,2-Dihydro-1,3,5-triazin-1-yl, 2H-1,2-Dihydro-1,2,4-triazin-1-yl, 2H-1,2,5,6-Tetrahydro-1,2,4-triazin-1-yl oder 4H-1,4,5,6-Tetrahydro-1,2,4-triazin-1-yl, bedeutet, und A durch Niederalkyl substituiertes geradkettiges Niederalkylen darstellt, von optischen Isomeren von Verbindungen der Formel I, von Mischungen dieser optischen Isomere und von Salzen, insbesondere pharmazeutisch annehmbaren Salzen von solchen Verbindungen der Formel I, die eine salzbildende Gruppe aufweisen.

4. Verfahren zur Herstellung von Verbindungen der Formel I gemäss Patentanspruch 1, worin $R_1$ durch Hydroxy substituiertes Niederalkyl ist, $R_2$ Carboxyl 1-Niederalkoxycarbonyloxyniederalkoxycarbonyl oder Niederalkanoyloxymethoxycarbonyl bedeutet, $R_3$ über ein tertiäres Stickstoffatom an den Rest -A- gebundenes Pyrrolyl, Imidazolyl, z.B. 1-Imidazolyl, 1H-Triazolyl, z.B. 1H-1,2,4-Triazol-1-yl, oder unsubstituiertes oder durch Amino, Carboxyniederalkyl, Sulfoniederalkyl oder Diniederalkylaminoniederalkyl substituiertes 1H-Tetrazolyl, z.B. 1H-Tetrazol-1-yl oder 2H-Tetrazol-2-yl, darstellt, und A durch Niederalkyl mit 1-2 Kohlenstoffatomen mono- oder di-substituiertes Niederalkylen mit 1-4 Kohlenstoffatomen bedeutet, von optischen Isomeren von Verbindungen der Formel I, z.B. den (5R,6S)-Isomeren, von Mischungen dieser optischen Isomere und von pharmazeutisch annehmbaren Salzen von solchen Verbindungen der Formel I, die eine salzbildende Gruppe aufweisen.

5. Verfahren zur Herstellung von (5R,6S)-konfigurierten Verbindungen der Formel I gemäss Patentanspruch 1, worin $R_1$ Hydroxymethyl oder 1-Hydroxyäthyl bedeutet, $R_2$ Carboxyl ist, $R_3$ Pyrrol-1-yl oder 1H-Tetrazol-1-yl darstellt und A 1,2-Propylen ist, und von pharmazeutisch annehmbaren Salzen davon.

6. Verfahren zur Herstellung von (5R,6S)-2-[(2R,S)-2-(Pyrrol-1-yl)-prop-1-yl]-6-hydroxymethyl-2-penem-3-carbonsäure und von pharmazeutisch annehmbaren Salzen davon, gemäss Patentanspruch 1.

7. Verfahren zur Herstellung von (5R,6S)-2-[(2R,S)-2-(Tetrazol)-1-yl)-prop-1-yl]-6-hydroxymethyl-2-penem-3-carbonsäure und von pharmazeutisch annehmbaren Salzen davon, gemäss Patentanspruch 1.

8. Verfahren zur Herstellung von pharmazeutischen Präparaten, dadurch gekennzeichnet, dass man eine nach dem Verfahren gemäss Anspruch 1 erhaltene Verbindung der Formel I oder ein pharmazeutisch annehmbares Salz einer solchen Verbindung mit einem pharmazeutisch annehmbaren Trägermaterial mischt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0125207
Nummer der Anmeldung

EP 84 81 0204

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE - A - 2 950 898 (BRISTOL-MYERS)<br>* Ansprüche 1-5,10,11 *<br>-- | 1,8,9,<br>11,12 | C 07 D 499/00<br>A 61 K 31/43//<br>C 07 F 7/18<br>C 07 D 257/04<br>C 07 D 205/08<br>C 07 F 9/65 |
| Y | EP - A - 0 003 960 (CIBA-GEIGY)<br>* Ansprüche 1-11 *<br>-- | 1,8,9,<br>11,12 | |
| Y | EP - A - 0 002 210 (MERCK)<br>* Ansprüche *<br>---- | 1,8,9,<br>11,12 | |

RECHERCHIERTE SACHGEBIETE (Int. Cl. 3)

C 07 D 499/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9,11-13
Unvollständig recherchierte Patentansprüche:
Nicht recherchierte Patentansprüche: 10
Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| Den Haag | 06-07-1984 | CHOULY |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82